# EUROPEAN PATENT SPECIFICATION

(11) **EP 3 870 295 B1**
(45) Date of publication and mention of the grant of the patent: **08.03.2023**
(21) Application number: 19795484.5
(22) Date of filing: 22.10.2019
(51) Int. Cl.: A61P 31/12, C07D 491/052, A61K 31/436, A61K 31/4725, A61K 31/444, A61K 31/407

(54) **NOVEL TRICYCLIC COMPOUNDS FOR THE TREATMENT AND PROPHYLAXIS OF HEPATITIS B VIRUS DISEASE**
NEUARTIGE TRICYCLISCHE VERBINDUNGEN ZUR BEHANDLUNG UND PROPHYLAXE VON HEPATITIS-B-VIRUS-INFEKTION
NOUVEAUX COMPOSÉS TRICYCLIQUES POUR LE TRAITEMENT ET LA PROPHYLAXIE D'UNE MALADIE DU VIRUS DE L'HÉPATITE B

(30) Priority: 24.10.2018 WO PCT/CN2018/111696
(43) Date of publication of application: 01.09.2021
(73) Proprietor: F. Hoffmann-La Roche AG, 4070 Basel (CH)
(72) Inventor: FENG, Song, Shanghai, 201203 (CN); JIANG, Min, Shanghai, 201203 (CN); TAN, Xuefei, Shanghai, 201203 (CN); WU, Jun, Shanghai, 201203 (CN)
(74) Representative: Jochnowitz, Evan
(86) International application number: PCT/EP2019/078620
(87) International publication number: WO 2020/083855

(56) References cited:
- WO-A1-2011/157682
- WO-A1-2017/202798
- WO-A2-2013/181584
- US-A- 4 377 518
- ANTHONY A EMANUELE ET AL: "Potential novel antibiotics from HTS targeting the virulence-regulating transcription factor, VirF, from Shigella flexneri", THE JOURNAL OF ANTIBIOTICS, vol. 67, no. 5, 1 May 2014 (2014-05-01), pages 379-386, XP055644529, GB ISSN: 0021-8820, DOI: 10.1038/ja.2014.10

## Description

The present invention relates to organic compounds useful for therapy and/or prophylaxis of HBV infection in a mammal, and in particular to cccDNA (covalently closed circular DNA) inhibitors useful for treating HBV infection.

### FIELD OF THE INVENTION

The present invention relates to novel tricyclic compounds having pharmaceutical activity, their manufacture, pharmaceutical compositions containing them and their potential use as medicaments.

The present invention relates to compounds of formula (I) 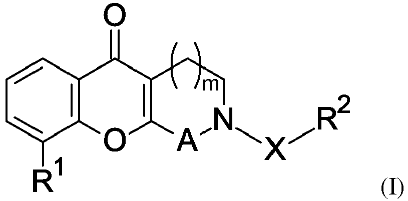 wherein R¹, R², A, X and m are as described below, or a pharmaceutically acceptable salt thereof.

Hepatitis B virus (HBV) infection is one of the most prevalent viral infections and is a leading cause of chronic hepatitis. It is estimated that worldwide, around 2 billion people have evidence of past or present infection with HBV. Over 250 million individuals are currently chronically infected with HBV and are therefore at high risk to develop liver fibrosis, cirrhosis and hepatocellular carcinoma (HCC). There are data to indicate ~800,000 deaths per year are directly linked to HBV infection (Lozano, R. et al., Lancet (2012), 380 (9859), 2095-2128; Goldstein, S.T. et al., Int J Epidemiol (2005), 34 (6), 1329-1339).

Many countries in the world administer hepatitis B vaccine starting at birth or in early childhood, which has greatly reduced the incidence and prevalence of hepatitis B in most endemic regions over the past few decades. However, the vaccine has no impact on people who were infected before the widely use of the vaccine in developing end-stage liver disease or HCC (Chen, D.S., J Hepatol (2009), 50 (4), 805-816). Vaccination at birth of infants born to HBV positive mothers is usually not sufficient for protecting vertical transmission and combination with hepatitis B immune globulin is needed (Li, X.M. et al., World J Gastroenterol (2003), 9 (7), 1501-1503).

Currently FDA-approved treatments for chronic hepatitis B include two type 1 interferons (IFN) which are IFNalfa-2b and pegylated IFN alfa-2a and six nucleos(t)ide analogues (NAs) which are lamivudine (3TC), tenofovir disoproxil fumarate (TDF), adefovir (ADV), telbivudine (LdT), entecavir (ETV), and vemlidy (tenofovir alafenamide (TAF)). IFN treatment is finite, but it is known to have severe side effects, and only a small percentage of patients showed a sustained virological response, measured as loss of hepatitis B surface antigen (HBsAg). NAs are inhibitors of the HBV reverse transcriptase, profoundly reduce the viral load in vast majority of treated patients, and lead to improvement of liver function and reduced incidence of liver failure and hepatocellular carcinoma. However, the treatment of NAs is infinite (Ahmed, M. et al., Drug Discov Today (2015), 20 (5), 548-561; Zoulim, F. and Locarnini, S., Gastroenterology (2009), 137 (5), 1593-1608 e1591-1592).

HBV chronic infection is caused by persistence of covalently closed circular (ccc)DNA, which exists as an episomal form in hepatocyte nuclei. cccDNA serves as the template for viral RNA transcription and subsequent viral DNA generation. Only a few copies of cccDNA per liver cell can establish or re-initiate viral replication. Therefore, a complete cure of chronic hepatitis B will require elimination of cccDNA or permanently silencing of cccDNA. However, cccDNA is intrinsically very stable and currently available therapeutics could not eliminate cccDNA or permanently silence cccDNA (Nassal, M., Gut (2015), 64 (12), 1972-1984; Gish, R.G. et al., Antiviral Res (2015), 121, 47-58; Levrero, M. et al., J Hepatol (2009), 51 (3), 581-592.). The current SoC could not eliminate the cccDNA which are already present in the infected cells. There is an urgent need to discover and develop new anti-HBV reagents to eliminate or permanently silence cccDNA, the source of chronicity (Ahmed, M. et al., Drug Discov Today (2015), 20 (5), 548-561; Nassal, M., Gut (2015), 64 (12), 1972-1984). Inhibitors of cccDNA for use in the treatment of HBV infections have been disclosed in WO 2013/181584

### SUMMARY OF THE INVENTION

Objects of the present invention are novel compounds of formula (I), their manufacture, medicaments based on a compound in accordance with the invention and their production as well as the use of compounds of formula (I) as cccDNA inhibitors and for the treatment or prophylaxis of HBV infection. The compounds of formula (I) show superior anti-HBV activity. In addition, the compounds of formula (I) also show good PK profiles.

The present invention relates to a compound of formula (I) 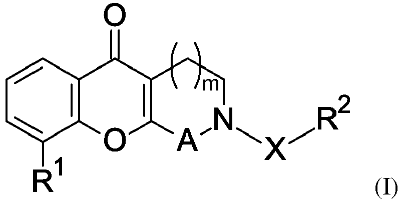 Wherein
- R¹: is halogen;
- R²: is C₁₋₆alkyl, phenyl, phenylC₁₋₆alkyl, pyrazolyl, pyridyl or isoquinolyl; wherein phenyl, phenylC₁₋₆alkyl, pyrazolyl and pyridyl are unsubstituted or substituted by one or two or three substituents independently selected from halogen, C₁₋₆alkyl, haloC₁₋₆alkyl, C₁₋₆alkoxy, haloC₁₋₆alkoxy, C₁₋₆alkoxyC₁₋₆alkyl, C₁₋₆alkoxyC₁₋₆alkoxy, C₁₋₆alkoxycarbonyl, C₁₋₆alkoxyphenylC₁₋₆alkoxy, carboxyC₃₋₇cycloalkoxy, CN and pyrrolidinylcarbonyl;
- X: is a bond, -C(O)-, -C(O)O- or -C(O)-NH-;
- A: is -C(R³R⁴)- or -C(O)-;
wherein
R³ is H or C₁₋₆alkyl;
R⁴ is H or C₁₋₆alkyl;
- m: is 0 or 1;
or a pharmaceutically acceptable salt thereof.

### DETAILED DESCRIPTION OF THE INVENTION

### DEFINITIONS

As used herein, the term "C₁₋₆alkyl" alone or in combination signifies a saturated, linear- or branched chain alkyl group containing 1 to 6, particularly 1 to 4 carbon atoms, for example methyl, ethyl, propyl, isopropyl, 1-butyl, 2-butyl, *tert*-butyl and the like. Particular "C₁₋₆alkyl" groups are methyl, ethyl, isopropyl and *tert*-butyl. More particularly, "C₁₋₆alkyl" group is methyl.

The term "C₁₋₆alkoxy" alone or in combination signifies a group C₁₋₆alkyl-O-, wherein the "C₁₋₆alkyl" is as defined above; for example methoxy, ethoxy, propoxy, *iso*-propoxy, *n*-butoxy, *iso*-butoxy, 2-butoxy, *tert*-butoxy, pentoxy, hexyloxy and the like. Particular "C₁₋₆alkoxy" groups are methoxy, ethoxy and propoxy. More particularly, "C₁₋₆alkoxy" group is methoxy or ethoxy.

The term "C₃₋₇cycloalkyl" denotes to a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular "C₃₋₇cycloalkyl" group is cyclopropyl, cyclobutyl or cyclopentyl.

The term "C₃₋₇cycloalkoxy" denotes a group C₃₋₇cycloalkyl-O-, wherein the "C₃₋₇cycloalkyl" is as defined above; for example cyclopropoxy, cyclobutoxy, cyclopentoxy. Particular "C₃₋₇cycloalkoxy" group is cyclobutoxy.

The term "halogen" and "halo" are used interchangeably herein and denote fluoro, chloro, bromo, or iodo.

The term "haloC₁₋₆alkyl" denotes an alkyl group wherein at least one of the hydrogen atoms of the alkyl group is replaced by same or different halogen atoms, particularly fluoro atoms. Examples of haloC₁₋₆alkyl include monochloro-, difluoro-or trifluoro-methyl, -ethyl or - propyl, for example difluoromethyl and trifluoromethyl.

The term "haloC₁₋₆alkoxy" denotes a C₁₋₆alkoxy group wherein at least one of the hydrogen atoms of the C₁₋₆alkoxy group is replaced by same or different halogen atoms, particularly fluoro atoms. Examples of haloC₁₋₆alkoxy include monofluoro-, difluoro- or trifluoro-methoxy, -ethoxy or -propoxy, for example trifluoromethoxy.

The term "carbonyl" alone or in combination refers to the group -C(O)-.

The term "bond" refers to a chemical bond between two atoms, or two moieties when the atoms joined by the bond are considered to be part of larger substructure. In one aspect, when a group described herein is a bond, the referenced group is absent thereby allowing a bond to be formed between the remaining identified groups.

The term "oxo" means an =O group and may be attached to a carbon atom or a sulfur atom.

The compounds according to the present invention may exist in the form of their pharmaceutically acceptable salts. The term "pharmaceutically acceptable salt" refers to conventional acid-addition salts or base-addition salts that retain the biological effectiveness and properties of the compounds of formula (I) and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as *p*-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin R.J., et al., Organic Process Research & Development 2000, 4, 427-435. Particular are the sodium salts of the compounds of formula (I).

Compounds of the general formula (I) which contain one or several chiral centers can either be present as racemates, diastereomeric mixtures, or optically active single isomers. The racemates can be separated according to known methods into the enantiomers. Particularly, diastereomeric salts which can be separated by crystallization are formed from the racemic mixtures by reaction with an optically active acid such as e.g. D- or L-tartaric acid, mandelic acid, malic acid, lactic acid or camphorsulfonic acid.

### cccDNA INHIBITORS

The present invention provides (i) a compound having the general formula (I): 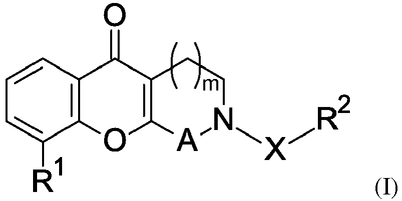
- R¹: is halogen;
- R²: is C₁₋₆alkyl, phenyl, phenylC₁₋₆alkyl, pyrazolyl, pyridyl or isoquinolyl; wherein phenyl, phenylC₁₋₆alkyl, pyrazolyl and pyridyl are unsubstituted or substituted by one or two or three substituents independently selected from halogen, C₁₋₆alkyl, haloC₁₋₆alkyl, C₁₋₆alkoxy, haloC₁₋₆alkoxy, C₁₋₆alkoxyC₁₋₆alkyl, C₁₋₆alkoxyC₁₋₆alkoxy, C₁₋₆alkoxycarbonyl, C₁₋₆alkoxyphenylC₁₋₆alkoxy, carboxyC₃₋₇cycloalkoxy, CN and pyrrolidinylcarbonyl;
- X: is a bond, -C(O)-, -C(O)O- or -C(O)-NH-;
- A: is -C(R³R⁴)- or -C(O)-;
wherein
R³ is H or C₁₋₆alkyl;
R⁴ is H or C₁₋₆alkyl;
- m: is 0 or 1;
or a pharmaceutically acceptable salt thereof.

A further embodiment of the present invention is (ii) a compound of formula (I) according to (i), wherein
- R¹: is Cl;
- R²: is butyl, phenyl, phenylmethyl, pyrazolyl, pyridyl or isoquinolyl; wherein phenyl, phenylmethyl, pyrazolyl and pyridyl are unsubstituted or substituted by one or two or three substituents independently selected from F, Cl, Br, CF₃, CN, methyl, methoxy, ethoxy, methoxymethyl, methoxymethoxy, methoxycarbonyl, trifluoromethoxy, carboxycyclobutoxy, pyrrolidinylcarbonyl and methoxyphenylmethoxy;
- X: is a bond, -C(O)-, -C(O)O- or -C(O)-NH-;
- A: is -C(R³R⁴)- or -C(O)-;
wherein
R³ is H or methyl;
R⁴ is H or methyl;
- m: is 0 or 1;
or a pharmaceutically acceptable salt thereof.

A further embodiment of the present invention is (iii) a compound of formula (I) according to (i), or a pharmaceutically acceptable salt thereof, wherein X is a bond.

A further embodiment of the present invention is (iv) a compound of formula (I) according to (i), or a pharmaceutically acceptable salt thereof, wherein A is -CH₂- or -C(O)-.

A further embodiment of the present invention is (v) a compound of formula (I) according to (i), or a pharmaceutically acceptable salt thereof, wherein R² is phenyl, which is unsubstituted or substituted by one or two substituents independently selected from halogen, haloC₁₋₆alkyl, C₁₋₆alkoxy, C₁₋₆alkoxycarbonyl.

A further embodiment of the present invention is (vi) a compound of formula (I) according to (i), or a pharmaceutically acceptable salt thereof, wherein R² is phenyl, which is unsubstituted or substituted by one or two substituents independently selected from F, Cl, CF₃, methoxy and methoxycarbonyl.

A further embodiment of the present invention is (vii) a compound of formula (I) according to (i), or a pharmaceutically acceptable salt thereof, wherein
- R¹: is halogen;
- R²: is phenyl, which is unsubstituted or substituted by one or two substituents independently selected from halogen, haloC₁₋₆alkyl, C₁₋₆alkoxy, C₁₋₆alkoxycarbonyl;
- X: is a bond;
- A: is -CH₂- or -C(O)-;
- m: is 0 or 1.

A further embodiment of the present invention is (viii) a compound of formula (I) according to (i), or a pharmaceutically acceptable salt thereof, wherein
- R¹: is Cl;
- R²: is phenyl, which is unsubstituted or substituted by one or two substituents independently selected from F, Cl, CF₃, methoxy and methoxycarbonyl.

In another embodiment (ix) of the present invention, particular compounds of the present invention are selected from:
*tert*-butyl 9-chloro-5-oxo-3,4-dihydro-1H-chromeno[2,3-c]pyridine-2(5H)-carboxylate;
2-benzyl-9-chloro-3,4-dihydro-1H-chromeno[2,3-c]pyridin-5(2H)-one;
methyl 3-((9-chloro-5-oxo-3,4-dihydro-1H-chromeno[2,3-c]pyridin-2(5H)-yl)methyl)benzoate;
9-chloro-2-(2-fluorobenzoyl)-3,4-dihydro-1H-chromeno[2,3-c]pyridin-5(2H)-one;
9-chloro-2-[(4-methoxyphenyl)methyl]-3,4-dihydrochromeno[2,3-c]pyridine-1,5-dione;
9-chloro-2-(isoquinoline-1-carbonyl)-3,4-dihydro-1H-chromeno[2,3-c]pyridin-5(2H)-one;
9-chloro-*N*-(2-fluorophenyl)-5-oxo-3,4-dihydro-1H-chromeno[2,3-c]pyridine-2(SH)-carboxamide;
methyl 3-(9-chloro-1,5-dioxo-3,4-dihydro-1H-chromeno[2,3-c]pyridin-2(SH)-yl)benzoate;
9-chloro-2-(4-methoxyphenyl)-3,4-dihydro-1H-chromeno[2,3-c]pyridine-1,5(2H)-dione;
methyl 4-(9-chloro-1,5-dioxo-3,4-dihydro-1H-chromeno[2,3-c]pyridin-2(SH)-yl)benzoate;
3-(4-(9-chloro-1,5-dioxo-3,4-dihydro-1H-chromeno[2,3-c]pyridin-2(5H)-yl)phenoxy)cyclobutanecarboxylic acid;
9-chloro-2-(4-chlorophenyl)-3,4-dihydro-1H-chromeno[2,3-c]pyridine-1,5(2H)-dione;
methyl 6-(9-chloro-5-oxo-3,4-dihydro-1H-chromeno[2,3-c]pyridin-2(5H)-yl)nicotinate;
9-chloro-2-(4-fluorophenyl)-3,4-dihydro-2H-chromeno[2,3-c]pyridine-1,5-dione;
2-(4-bromophenyl)-9-chloro-3,4-dihydro-2H-chromeno[2,3-c]pyridine-1,5-dione;
9-chloro-2-(6-chloropyridin-3-yl)-3,4-dihydro-2H-chromeno[2,3-c]pyridine- 1,5-dione;
9-chloro-2-(4-chloro-2-methoxyphenyl)-3,4-dihydro-2H-chromeno[2,3-c]pyridine-1,5-dione;
9-chloro-2-(4-fluoro-3-(trifluoromethyl)phenyl)-3,4-dihydro-2H-chromeno[2,3-c]pyridine-1,5-dione;
9-chloro-2-phenyl-3,4-dihydro-2H-chromeno[2,3-c]pyridine-1,5-dione;
9-chloro-2-(4-(trifluoromethyl)phenyl)-3,4-dihydro-2H-chromeno[2,3-c]pyridine-1,5-dione;
9-chloro-2-(3-(trifluoromethoxy)phenyl)-3,4-dihydro-2H-chromeno[2,3-c]pyridine-1,5-dione;
9-chloro-2-(4-chloro-3-(trifluoromethyl)phenyl)-3,4-dihydro-2H-chromeno [2,3-c]pyridine-1,5-dione;
9-chloro-2-(2-(trifluoromethoxy)phenyl)-3,4-dihydro-2H-chromeno[2,3-c]pyridine-1,5-dione;
methyl 2-chloro-5-(9-chloro-1,5-dioxo-1,3,4,5-tetrahydro-2H-chromeno[2,3-c]pyridin-2-yl)benzoate;
3-(2-(9-chloro-1,5-dioxo-1,3,4,5-tetrahydro-2H-chromeno[2,3-c]pyridin-2-yl)phenoxy)cyclobutane-1-carboxylic acid;
3-(5-chloro-2-(9-chloro-1,5-dioxo-1,3,4,5-tetrahydro-2H-chromeno[2,3-c]pyridin-2-yl)phenoxy)cyclobutane-1-carboxylic acid;
9-chloro-2-(4-chloro-2-(trifluoromethyl)phenyl)-3,4-dihydro-2H-chromeno[2,3-c]pyridine-1,5-dione;
9-chloro-2-(3,4-dichlorophenyl)-3,4-dihydro-2H-chromeno[2,3-c]pyridine- 1,5-dione;
9-chloro-2-(4-chloro-2-fluorophenyl)-3,4-dihydro-2H-chromeno[2,3-c]pyridine- 1,5-dione;
4- (9-chloro- 1,5-dioxo-1,3,4,5-tetrahydro-2H-chromeno[2,3-c]pyridin-2-yl)benzonitrile;
9-chloro-2-(2,5-dimethylphenyl)-3,4-dihydro-2H-chromeno[2,3-c]pyridine-1,5-dione;
9-chloro-2-(2-chloro-4-(trifluoromethoxy)phenyl)-3,4-dihydro-2H-chromeno[2,3-c]pyridine-1,5-dione;
9-chloro-2-(4-(trifluoromethoxy)phenyl)-3,4-dihydro-2H-chromeno[2,3-c]pyridine-1,5-dione;
9-chloro-2-(p-tolyl)-3,4-dihydro-2H-chromeno[2,3-c]pyridine-1,5-dione;
9-chloro-2-(1-methyl-1H-pyrazol-4-yl)-3,4-dihydro-2H-chromeno[2,3-c]pyridine-1,5-dione;
methyl 5-chloro-2-(9-chloro-1,5-dioxo-1,3,4,5-tetrahydro-2H-chromeno[2,3-c]pyridin-2-yl)benzoate;
5-chloro-2-(9-chloro-1,5-dioxo-1,3,4,5-tetrahydro-2H-chromeno[2,3-c]pyridin-2-yl)benzonitrile;
9-chloro-2-(4-chloro-2-methylphenyl)-3,4-dihydro-2H-chromeno[2,3-c]pyridine-1,5-dione;
9-chloro-2-(4-(trifluoromethyl)phenyl)-1,2,3,4-tetrahydro-5H-chromeno[2,3-c]pyridin-5-one;
9-chloro-2-(4-methoxyphenyl)-1,2,3,4-tetrahydro-5H-chromeno[2,3-c]pyridin-5-one;
9-chloro-2-(3-methoxyphenyl)-1,2,3,4-tetrahydro-5H-chromeno[2,3-c]pyridin-5-one;
9-chloro-2-(4-chlorophenyl)-1,2,3,4-tetrahydro-5H-chromeno[2,3-c]pyridin-5-one;
9-chloro-2-(5-chloro-3-(trifluoromethyl)pyridin-2-yl)-3,4-dihydro-2H-chromeno[2,3-c]pyridine-1,5-dione;
2-(2-bromo-4-chlorophenyl)-9-chloro-3,4-dihydro-2H-chromeno[2,3-c]pyridine-1,5-dione;
9-chloro-2-(5-chloro-3-((4-methoxybenzyl)oxy)pyridin-2-yl)-3,4-dihydro-2H-chromeno[2,3-c]pyridine-1,5-dione;
9-chloro-2-(4-chloro-2-(methoxymethoxy)phenyl)-3,4-dihydro-2H-chromeno[2,3-c]pyridine-1,5-dione;
9-chloro-2-(4-chloro-2-(pyrrolidine-1-carbonyl)phenyl)-3,4-dihydro-2H-chromeno[2,3-c]pyridine-1,5-dione;
9-chloro-2-(4-chloro-2-ethoxyphenyl)-3,4-dihydro-2H-chromeno[2,3-c]pyridine-1,5-dione;
9-chloro-2-(3,4-dichlorophenyl)-1,2,3,4-tetrahydro-5H-chromeno[2,3-c]pyridin-5-one;
9-chloro-2-(4-chloro-2-(methoxymethyl)phenyl)-3,4-dihydro-2H-chromeno[2,3-c]pyridine-1,5-dione;
5-chloro-2-(4-chlorophenyl)-1H-chromeno[2,3-c]pyrrole-3,9-dione; and
5-chloro-2-(4-methoxyphenyl)-1H-chromeno[2,3-c]pyrrole-3,9-dione;
or a pharmaceutically acceptable salt thereof.

In another embodiment (x) of the present invention, particular compounds of the present invention are selected from:
methyl 3-(9-chloro-1,5-dioxo-3,4-dihydro-1H-chromeno[2,3-c]pyridin-2(5H)-yl)benzoate;
9-chloro-2-(4-methoxyphenyl)-3,4-dihydro-1H-chromeno[2,3-c]pyridine-1,5(2H)-dione;
9-chloro-2-(4-chlorophenyl)-3,4-dihydro-1H-chromeno[2,3-c]pyridine-1,5(2H)-dione;
9-chloro-2-(4-fluorophenyl)-3,4-dihydro-2H-chromeno[2,3-c]pyridine-1,5-dione;
9-chloro-2-(4-(trifluoromethyl)phenyl)-3,4-dihydro-2H-chromeno[2,3-c]pyridine-1,5-dione;
9-chloro-2-(3,4-dichlorophenyl)-3,4-dihydro-2H-chromeno[2,3-c]pyridine- 1,5-dione;
9-chloro-2-(4-chloro-2-fluorophenyl)-3,4-dihydro-2H-chromeno[2,3-c]pyridine-1,5-dione;
9-chloro-2-(4-(trifluoromethyl)phenyl)-1,2,3,4-tetrahydro-5H-chromeno[2,3-c]pyridin-5-one; and
5-chloro-2-(4-chlorophenyl)-1H-chromeno[2,3-c]pyrrole-3,9-dione;
or a pharmaceutically acceptable salt thereof.

### SYNTHESIS

The compounds of the present invention can be prepared by any conventional means. Suitable processes for synthesizing these compounds as well as their starting materials are provided in the schemes below and in the subsequent examples. All substituents, in particular, R¹ to R⁴, A, X and m are defined above unless otherwise indicated. Furthermore, and unless explicitly otherwise stated, all reactions, reaction conditions, abbreviations and symbols have the meanings well known to a person of ordinary skill in the art. 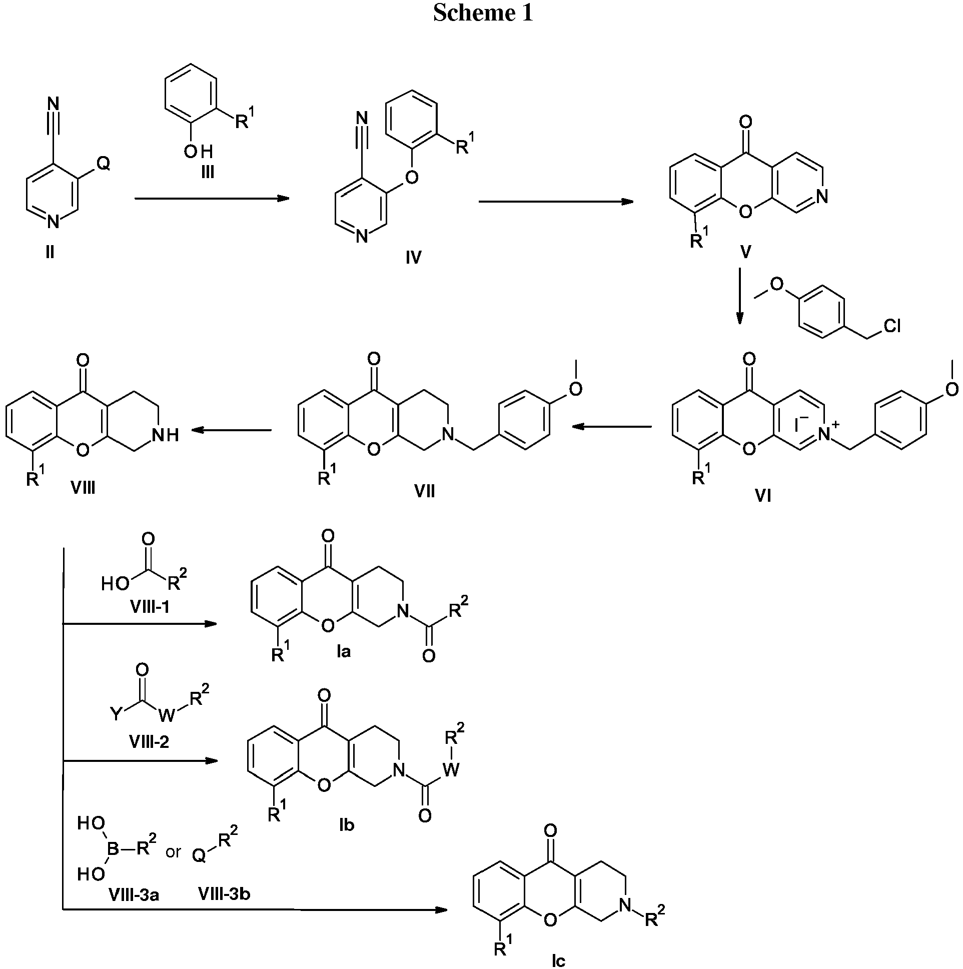 wherein Y is Cl or OC(O)WR²; Q is halogen, OTs, OTf or OMs; W is NH or O.

Substitution of compound of formula **II** with compound of formula **III** in the presence of a suitable base, such as Cs₂CO₃, in a suitable solvent, such as DMF, affords compound of formula **IV.** Cyclization of compound of formula **IV** in the presence of a suitable Lewis acid, such as PPA, affords compound of formula **V.** Quaternization of compound of formula **V** in the presence of 4-Methoxybenzyl chloride and KI, in a suitable solvent, such as MeCN, affords compound of formula **VI.** Reduction of compound of formula **VI** in the presence of a suitable reducer, such as NaBH₃CN, and a suitable catalyst, such as AcOH, in a suitable solvent, such as ethanol, affords compound of formula **VII.** Deprotection of compound of formula **VII** in the presence of a suitable reagent, such as 1-chloroethyl carbonochloridate, in a suitable solvent, such as DCE, affords compound of formula **VIII.** Condensation of compound of formula **VIII** with compound of formula **VIII-1** in the presence of a suitable condensation reagent, such as HATU, and a suitable base, such as DIEA, in a suitable solvent, such as DCM, affords compound of formula **Ia.**

The compound of formula **Ib** can be prepared by starting with condensation of compound of formula **VIII** with compound of formula **VIII-2** in the presence of a suitable base, such as TEA or DIEA, in a suitable solvent, such as DCM or MeOH, affords compound of formula **Ib.**

The compound of formula **Ic** can be prepared by starting with condensation compound of formula **VIII** with compound of formula **VIII-3a** in the presence of a suitable catalyst, such as Cu(OAc)₂, and a suitable base, such as TEA, in a suitable solvent, such as DCM, affords compound of formula **Ic.** The compound of formula **Ic** can also be prepared by condensation of formula **VIII** with compound of formula **VIII-3b** in the presence of a suitable base, such as DIEA or K₂CO₃, in a suitable solvent, such as DMF, affords compound of formula **Ic.** 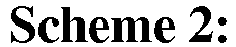 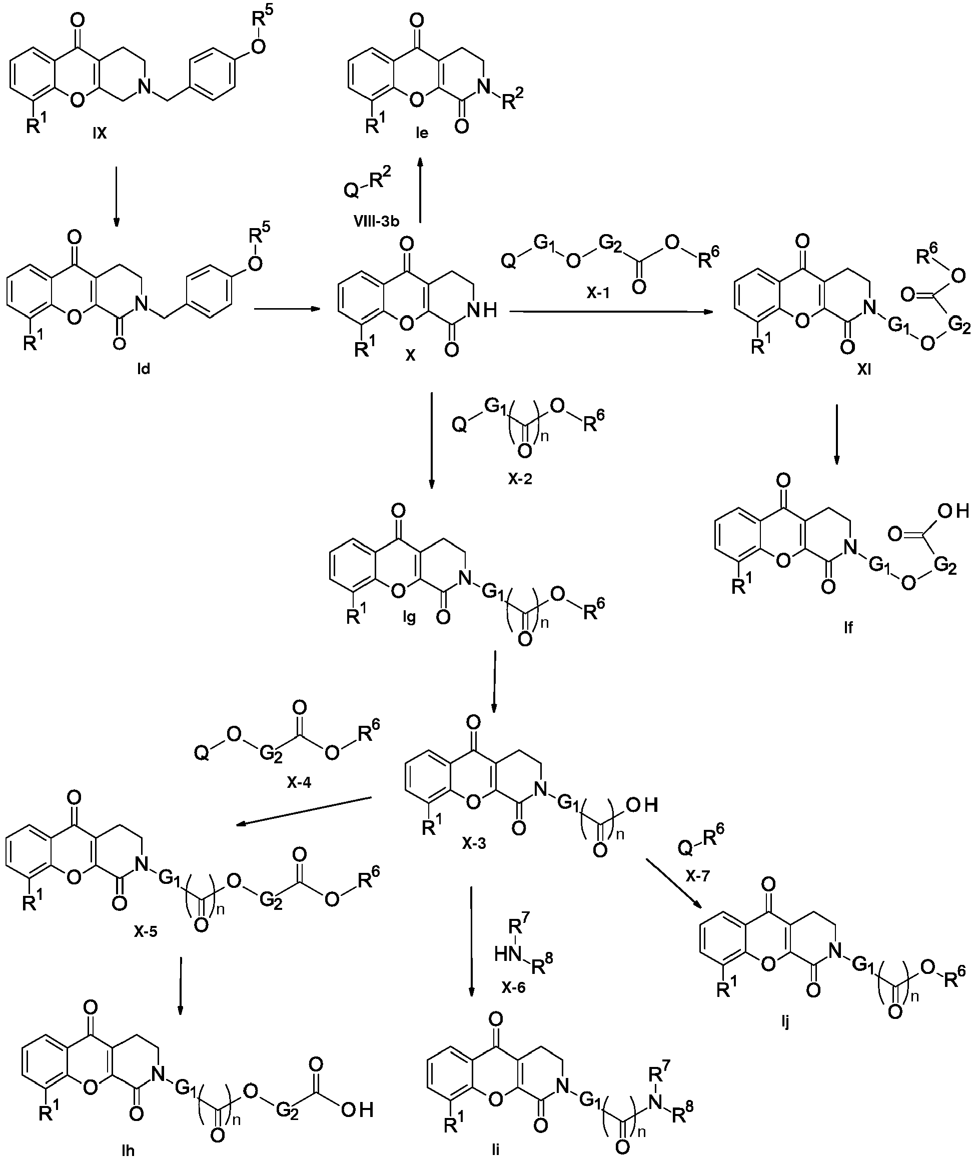 wherein G₁ is phenyl, which is unsubstituted or substituted by halogen; G₂ is C₃₋₇cycloalkyl; R⁵ is C₁₋₆alkyl; R⁶ is C₁₋₆alkyl; R⁷ and R⁸, together with the atom to which they are attached, form a ring; Q is halogen, OTs, OTf or OMs; n is 0 or 1.

Oxidation of compound of formula **IX** in the presence of an appropriate oxidant, such as iodine, and a suitable base, such as NaHCOs, in a suitable solvent, such as THF and water, affords compound of formula **Id.** Deprotection of compound of formula **Id** in the presence of a suitable Lewis acid, such as TFA, affords compound of formula **X.** Condensation of **X** with compound of formula **VIII-3b** in the presence of a suitable catalyst, such as CuI, and a suitable base, such as Cs₂CO₃ or K₃PO₄, in a suitable solvent, such as DMF, affords compound of formula **Ie.**

The compound of formula **If** can be prepared by starting with coupling of compound of formula **X** with compound of formula **X-1** in the presence of a suitable catalyst, such as CuI, and a suitable base, such as Cs₂CO₃, in a suitable solvent, such as DMF, affords compound of formula **XI.** Hydrolysis of compound of formula **XI** in the presence of a suitable acid, such as hydrochloride acid, in a suitable solvent, such as water and THF, affords compound of formula **If.**

The compound of formula **Ih** can be prepared by starting with coupling of compound of formula **X** with compound of formula **X-2** in the presence of a suitable catalyst, such as CuI, and a suitable base, such as Cs₂CO₃, in a suitable solvent, such as DMF, affords compound of formula **Ig.** Hydrolysis of compound of formula **Ig** in the presence of a suitable Lewis acid, such as hydrochloride acid or BBr₃, in a suitable solvent, such as water with/without THF or DCM, affords compound of formula **X-3.** Coupling of compound of formula **X-3** with compound of formula **X-4** in the presence of suitable base, such as K₂CO₃, in a suitable solvent, such as DMF, affords compound of formula **X-5.** Hydrolysis of compound of formula **X-5** in the presence of suitable acid, such as hydrochloride acid, in a suitable solvent, such as water and THF, affords compound of formula **Ih.**

The compound of formula **Ii** can be prepared by starting with condensation of compound of formula **X-3** with compound of formula **X-6** in the presence of a suitable condensation reagent, such as HATU, a suitable base, such as DIEA, in a suitable solvent, such as DCM, affords compound of formula **Ii.**

The compound of formula **Ij** can be prepared by starting with condensation of **X-3** with compound of formula **X-7** in the presence of suitable base, such as Cs₂CO₃, in a suitable solvent, such as DMF, affords compound of formula **Ij.**

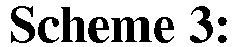

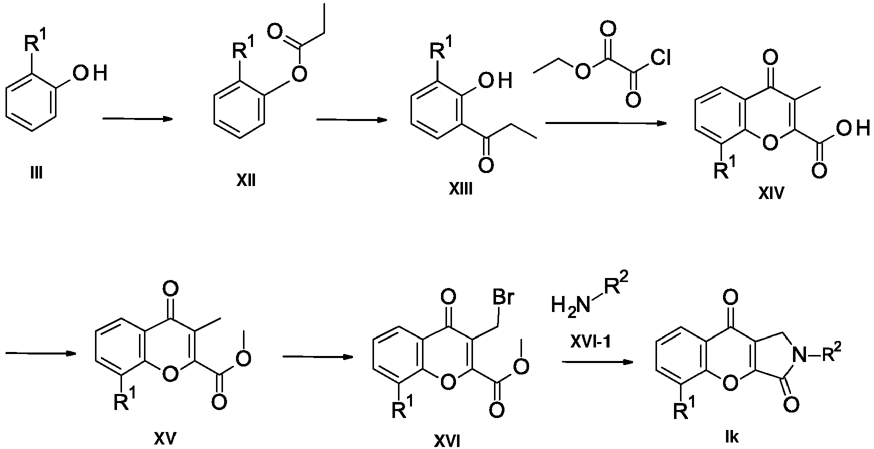

Acylation of compound of formula **III** with propanoyl chloride in the presence of a suitable base, such as TEA, in a suitable solvent, such as DCM, affords compound of formula **XII.** Treatment of intermediate **XII** with a suitable Lewis acid, such as AlCl₃, affords compound of formula **XIII.** Cyclization of compound of formula **XIII** with ethyl oxalyl chloride in the presence of a suitable base, such as pyridine, affords compound of formula **XIV.** Esterification of compound of formula **XIV** in the presence of a suitable Lewis acid, such as hydrochloride acid, in a suitable solvent, such as methanol, affords compound of formula **XV.** Bromination of compound of formula **XV** in the presence of a suitable bromination reagent, such as NBS, and a suitable evacator, such as BPO, in a suitable solvent such as CCl₄, affords compound of formula **XVI.** Cyclization of compound of formula **XVI** with **XVI-1** in a suitable solvent, such as EtOH, affords compound of formula **Ik.**

This invention also relates to a process for the preparation of a compound of formula (I) comprising any one of the following steps:
(a) Condensation of compound of formula (VIII), 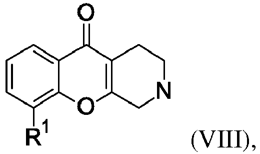 with compound of formula (VIII-1) in the presence of a condensation reagent and a base;
(b) Condensation of compound of formula (VIII) with compound of formula (VIII-2) in the presence of a base;
(c) Condensation of compound of formula (VIII) with compound of formula (VIII-3a) in the presence of a catalyst and a base;
(d) Condensation of compound of formula (VIII) with compound of formula (VIII-3b) in the presence of a base;
(e) Oxidation of compound of formula (IX), 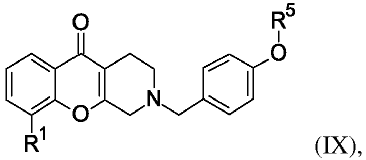 in the presence of an oxidant and a base;
(f) Condensation of compound of formula (X), 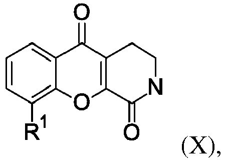 with compound of formula (VIII-3b) in the presence of a catalyst and a base;
(g) Hydrolysis of compound of formula (XI), 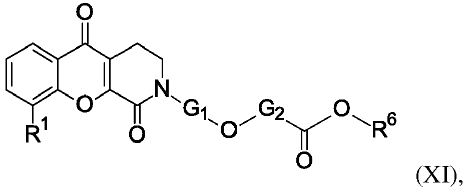 in the presence of an acid;
(h) Hydrolysis of compound of formula (X-5), 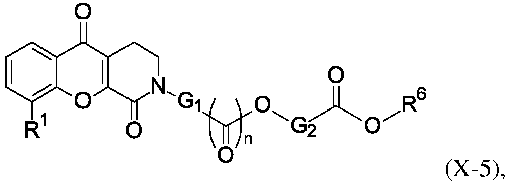 in the presence of an acid;
(i) Condensation of compound of formula (X-3), 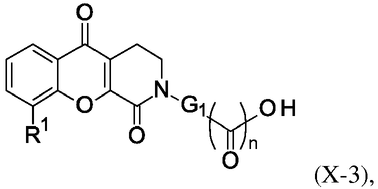 with compound of formula (X-6) in the presence of a condensation reagent and a base;
(j) Condensation of compound of formula (X-3) with compound of formula (X-7) in the presence of a base;
(k) Cyclization of compound of formula (XVI), 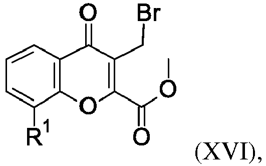 with compound of formula (XVI-1);
   the condensation reagent in step (a) and step (i), can be for example HATU;
   the base in step (a) and step (i), can be for example DIEA;
   the base in step (b), can be for example TEA or DIEA;
   the catalyst in step (c), can be for example Cu(OAc)₂;
   the base in step (c), can be for example TEA;
   the base in step (d), can be for example DIEA or K₂CO₃;
   the oxidant in step (e), can be for example iodine;
   the base in step (e), can be for example NaHCO₃;
   the catalyst in step (f), can be for example CuI;
   the base in step (f), can be for example Cs₂CO₃ or K₃PO₄;
   the acid in step (g) and step (h), can be for example hydrochloride;
   the base in step (j), can be for example Cs₂CO₃.

A compound of formula (I) when manufactured according to the above process is also an object of the invention.

### PHARMACEUTICAL COMPOSITIONS AND ADMINISTRATION

The invention also relates to a compound of formula (I) for use as therapeutically active substance. Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to inhibit cccDNA in HBV patients, consequently lead to the reduction of HBsAg and HBeAg (HBV e antigen) in serum. For example, such amount may be below the amount that is toxic to normal cells, or the mammal as a whole.

In one example, the pharmaceutically effective amount of the compound of the invention administered parenterally per dose will be in the range of about 0.1 to 100 mg/kg, alternatively about 0.1 to 50 mg/kg of patient body weight per day, with the typical initial range of compound used being 0.3 to 15 mg/kg/day. In another embodiment, oral unit dosage forms, such as tablets and capsules, preferably contain from about 25 to about 1000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 25 to 500 mg of the compound of the invention compounded with about 90 to 30 mg anhydrous lactose, about 5 to 40 mg sodium croscarmellose, about 5 to 30 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 400 mg, of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of Formula (I), or a pharmaceutically acceptable salt thereof.

In a further embodiment includes a pharmaceutical composition comprising a compound of formula (I), or a pharmaceutically acceptable salt thereof, together with a pharmaceutically acceptable carrier or excipient.

Another embodiment includes a pharmaceutical composition comprising a compound of formula (I), or a pharmaceutically acceptable salt thereof for use in the treatment of HBV infection.

### INDICATIONS AND METHODS OF TREATMENT

The compounds of the invention can inhibit cccDNA and have anti-HBV activity. Accordingly, the compounds of the invention are useful for the treatment or prophylaxis of HBV infection.

The invention relates to the use of a compound of formula (I) for the inhibition of cccDNA.

The invention also relates to the use of a compound of formula (I) for the inhibition of HBeAg.

The invention further relates to the use of a compound of formula (I) for the inhibition of HBsAg.

The invention relates to the use of a compound of formula (I) for the inhibition of HBV DNA.

The invention relates to the use of a compound of formula (I) for the treatment or prophylaxis of HBV infection.

The use of a compound of formula (I) for the preparation of medicaments useful in the treatment or prophylaxis diseases that are related to HBV infection is an object of the invention.

The invention relates in particular to the use of a compound of formula (I) for the preparation of a medicament for the treatment or prophylaxis of HBV infection.

Another embodiment includes a method for the treatment or prophylaxis of HBV infection, which method comprises administering an effective amount of a compound of Formula (I), or enantiomers, diastereomers, prodrugs or pharmaceutically acceptable salts thereof.

### EXAMPLES

The invention will be more fully understood by reference to the following examples. They should not, however, be construed as limiting the scope of the invention.

Abbreviations used herein are as follows:
- MeCN:: acetonitrile
- BBr₃:: boron tribromide
- DCE:: 1,2-dichloroethane
- DMF:: *N,N*-dimethylformamide
- EC₅₀:: the molar concentration of an inhibitor, which produces 50% of the maximum possible response for that inhibitor.
- FBS:: fetal bovine serum
- HPLC:: high performance liquid chromatography
- MS (ESI):: mass spectroscopy (electron spray ionization)
- obsd.:: observed
- PE:: petroleum ether
- EA:: ethyl acetate
- PPA:: polyphosphoric acid
- rt:: room temperature
- TFA:: trifluoroacetic acid
- DCM:: dichloromethane
- NBS:: *N*-bromosuccinimide
- MeOH:: methanol
- THF:: tetrahydrofuran
- BPO:: benzoyl peroxide
- HATU:: (1-[bis(dimethylamino)methylene]-1H-1,2,3-triazolo[4,5-b]pyridinium 3-oxid hexafluorophosphate
- DIEA:: *N*,*N*-diisopropylethylamine
- TEA:: triethylamine
- AcOH:: acetic acid
- δ:: chemical shift

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel Brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp C₁₈ (5 µm, OBD^{™} 30 × 100 mm) column or SunFire^{™} Perp C₁₈ (5 µm, OBD^{™} 30 × 100 mm) column.

LC/MS spectra were obtained using a Waters UPLC-SQD Mass. Standard LC/MS conditions were as follows (running time 3 minutes):
Acidic condition: A: 0.1% formic acid and 1% acetonitrile in H₂O; B: 0.1% formic acid in acetonitrile;
Basic condition: A: 0.05% NH₃·H₂O in H₂O; B: acetonitrile.

Mass spectra (MS): generally only ions which indicate the parent mass are reported, and unless otherwise stated the mass ion quoted is the positive mass ion (M+H)⁺.

NMR Spectra were obtained using Bruker Avance 400MHz.

All reactions involving air-sensitive reagents were performed under an argon atmosphere. Reagents were used as received from commercial suppliers without further purification unless otherwise noted.

### PREPARATIVE EXAMPLES

### Intermediate 1: 9-chloro-1,2,3,4-tetrahydrochromeno[2,3-c]pyridin-5-one; hydrochloride

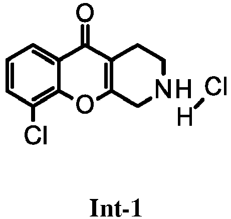

### Step 1: Preparation of 3-(2-chlorophenoxy)pyridine-4-carbonitrile

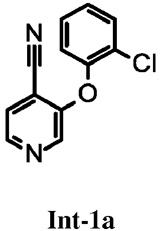

A mixture of 3-chloropyridine-4-carbonitrile (4 g, 28.9 mmol), 2-chlorophenol (4.45 g, 34.6 mmol) and Cs₂CO₃ (18.8 g, 57.7 mmol) in DMF (100 mL) was heated at 110°C for 2 hours. After the reaction was completed, the reaction was quenched with water. The resulting mixture was extracted with DCM (50 mL) three times. The combined organic layer was dried over anhydrous Na₂SO₄ and concentrated *in vacuo.* The residue was purified by silica-gel chromatography (elution with EA : PE = 10%) to afford desired product 3-(2-chlorophenoxy)pyridine-4-carbonitrile (6.3 g, 94%) as a yellow oil. MS obsd. (ESI⁺) [(M+H)⁺]: 231.1.

### Step 2: Preparation of 9-chlorochromeno[2,3-c]pyridin-5-one

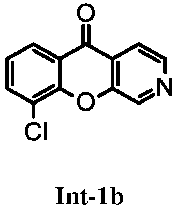

A solution of 3-(2-chlorophenoxy)pyridine-4-carbonitrile (5 g, 21.7 mmol) in PPA (40 g) was stirred at 220 °C for 0.5 hour. And then, the mixture was poured into ice-water (200 mL) and neutralized with NaOH solid. The resulting mixture was extracted with DCM (100mL) three times. The combined organic layer was dried over anhydrous Na₂SO₄ and concentrated *in vacuo.* The residue was purified by silical-gel chromatography (elution with MeOH: DCM = 8%) to afford 9-chlorochromeno[2,3-c]pyridin-5-one (2 g, 40%) as a white solid. MS obsd. (ESI⁺) [(M+H)⁺]: 232.1.

### Step 3: Preparation of 9-chloro-2-(4-methoxybenzyl)-5-oxo-SH-chromeno[2,3-c]pyridin-2-ium iodide

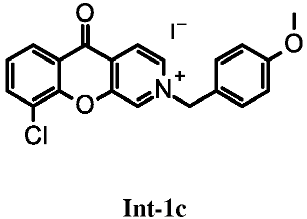

A mixture of 9-chlorochromeno[2,3-c]pyridin-5-one (1 g, 4.32 mmol), KI (1.6g, 9.64 mmol) and 1-(chloromethyl)-4-methoxybenzene (1.01 g, 6.48 mmol) in MeCN (30 mL) was heated at 80 °C for 5 hours to yield a suspension. And then, the solid was collected by filtration to afford 9-chloro-2-(4-methoxybenzyl)-5-oxo-5H-chromeno[2,3-c]pyridin-2-ium iodide (2.0 g, 97%). MS obsd. (ESI⁺) [(M)⁺]: 352.1.

### Step 4: Preparation of 9-chloro-2-[(4-methoxyphenyl)methyl]-3,4-dihydro-1H-chromeno[2,3-c]pyridin-5-one

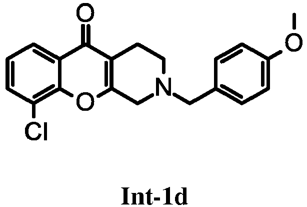

A mixture of 9-chloro-2-(4-methoxybenzyl)-5-oxo-5H-chromeno[2,3-c]pyridin-2-ium iodide (5.6 g, 11.7 mmol), NaBH₃CN (5.87 g, 93.4 mmol) and AcOH (5 mL, 11.7 mmol) in ethanol (40 mL) was stirred at rt for 1 hour. And then, the mixture was quenched with water (100 mL). The resulting mixture was extracted with DCM (50 mL) three times. The combined organic layer was dried over anhydrous Na₂SO₄ and concentrated *in vacuo.* The residue was purified by silica-gel chromatography (elution with MeOH: DCM = 10%) to afford 9-chloro-2-(4-methoxybenzyl)-3,4-dihydro-1H-chromeno[2,3-c]pyridin-5(2H)-one (3.1 g, 74%). MS obsd. (ESI⁺) [(M+H)⁺]: 356.2.

### Step 5: Preparation of 9-chloro-1,2,3,4-tetrahydrochromeno[2,3-c]pyridin-5-one hydrochloride

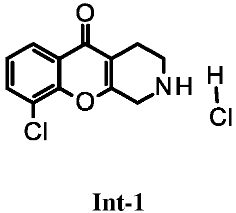

A mixture of 9-chloro-2-(4-methoxybenzyl)-3,4-dihydro-1H-chromeno[2,3-c]pyridin-5(2H)-one (1.4g, 3.93 mmol) and 1-chloroethyl carbonochloridate (3.0 g, 21.0 mmol) in DCE (40 mL) was heated at 110 °C for 10 hours. After removing the excess solvent, the residue was dissolved in MeOH (40 mL). To the resulting solution was added acetyl chloride (5 mL). The resulting mixture was heated at 76 °C for 1 hour. After removing the excess solvent, the residue was washed with diethyl ether (5 mL) to afford 9-chloro-1,2,3,4-tetrahydrochromeno[2,3-c]pyridin-5-one; hydrochloride (1.0 g, 93%) as a yellow solid. MS obsd. (ESI⁺) [(M+H)⁺]: 236.1.

### Intermediate 2: 9-chloro-3,4-dihydro-2H-chromeno[2,3-c]pyridine-1,5-dione

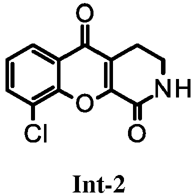

### Step 1: Preparation of 9-chloro-2-[(4-methoxyphenyl)methyl]-3,4-dihydrochromeno[2,3-c]pyridine-1,5-dione

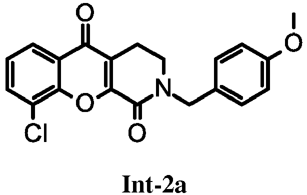

A mixture of 9-chloro-2-(4-methoxybenzyl)-3,4-dihydro-1H-chromeno[2,3-c]pyridin-5(2H)-one (3g, 8.43 mmol), iodine (6.28 g, 49.4 mmol) and NaHCO₃ (8.08 g, 96.2 mmol) in THF (50 ml) and water (50 mL) was heated at 60 °C for 16 hours. After the reaction was completed, the resulting mixture was diluted with DCM (150 mL) and washed with saturated Na₂SO₃ aqueous (100 mL). The organic layer was dried over anhydrous Na₂SO₄ and concentrated *in vacuo.* The residue was purified by silica-gel chromatography (elution with EA: PE = 40%) to afford 9-chloro-2-[(4-methoxyphenyl)methyl]-3,4-dihydrochromeno[2,3-c]pyridine-1,5-dione (1.4g, 45%). MS obsd. (ESI⁺) [(M+H)⁺]: 370.2.

### Step 2: Preparation of 9-chloro-3,4-dihydro-2H-chromeno[2,3-c]pyridine-1,5-dione

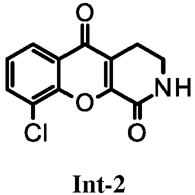

A solution of 9-chloro-2-[(4-methoxyphenyl)methyl]-3,4-dihydrochromeno[2,3-c]pyridine-1,5-dione (1.9g, 5.14 mmol) in TFA (25 mL) was heated at 90 °C for 16 hours. And then, the resulting mixture was evaporated *in vacuo* and the residue was dissolved in DCM (100 mL) and washed with saturated NaHCO₃ aqueous (100 mL). The organic layer was dried over anhydrous Na₂SO₄ and concentrated *in vacuo.* The residue was purified by silica-gel chromatography (elution with EA: PE = 80%) to afford 9-chloro-3,4-dihydro-2H-chromeno[2,3-c]pyridine-1,5-dione (1.0g, 78 %) as a grey solid. MS obsd. (ESI⁺) [(M+H)⁺]: 250.2.

### Intermediate 3: methyl 3-(bromomethyl)-8-chloro-4-oxo-chromene-2-carboxylate

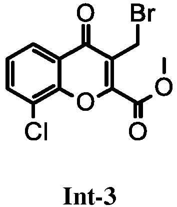

### Step 1: Preparation of (2-chlorophenyl) propanoate

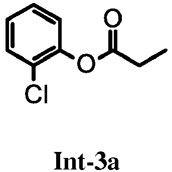

A mixture of 2-chlorophenol (12.1 mL, 116.7 mmol) and propanoyl chloride (11.9 g, 128.3 mmol), TEA (32.5 mL, 233.4 mmol) in DCM (200 mL) was stirred at 20 °C for 1 hour. And then, the resulting mixture was poured into water (100 mL) and extracted with DCM (100 mL) three times. The combined organic layer was concentrated *in vacuo.* The residue was purified by silica-gel chromatography (elution with EA: PE = 5%) to give the (2-chlorophenyl) propanoate (20.0 g, 91%) as a colorless liquid. MS obsd. (ESI⁺) [(M+H)⁺]: 185.1.

### Step 2: Preparation of 1-(3-chloro-2-hydroxy-phenyl)propan-1-one

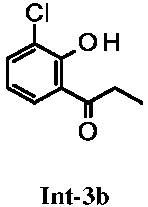

A mixture of (2-chlorophenyl) propanoate (15.0 g, 81.3 mmol) and aluminum chloride (10.8 g, 81.3 mmol) was heated at 180 °C for 2 hours. After cooling to rt, the resulting mixture was poured into 1N HCl aqueous (100 mL) and extracted with EA (100 mL) three times. The combined organic layer was concentrated to give the 1-(3-chloro-2-hydroxy-phenyl)propan-1-one (14.0 g, 93%) as a brown oil. MS obsd. (ESI⁺) [(M+H)⁺]: 185.1.

### Step 3: Preparation of 8-chloro-3-methyl-4-oxo-chromene-2-carboxylic acid

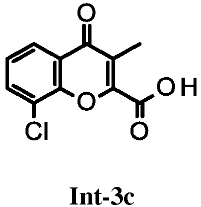

A mixture of 1-(3-chloro-2-hydroxy-phenyl)propan-1-one (15.0 g, 81.3 mmol) and ethyl oxalyl chloride (22.2 g, 162.5 mmol) in pyridine (30 mL) was stirred at 120 °C for 12 hours. After the reaction was completed, the resulting mixture was poured into the water (60 mL) and washed with EA (300 mL) three times. And then, the water layer was adjusted to pH = 2 and extracted with EA (300 mL) three times. The combined organic layer was concentrated to give the 8-chloro-3-methyl-4-oxo-chromene-2-carboxylic acid (18.0 g, 27%) as a brown oil. MS obsd. (ESI⁺) [(M+H)⁺]: 239.1.

### Step 4: Preparation of methyl 8-chloro-3-methyl-4-oxo-chromene-2-carboxylate

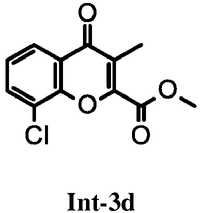

A mixture of 8-chloro-3-methyl-4-oxo-chromene-2-carboxylic acid (17.0 g, 71.2 mmol) in HCl solution (4 N in MeOH, 100 mL) was stirred at 20 °C for 24 hours. The resulting mixture was concentrated *in vacuo.* The residue was purified by silica-gel chromatography (elution with EA: PE = 10%) to give the methyl 8-chloro-3-methyl-4-oxo-chromene-2-carboxylate (2 g, 14%) as a white solid. MS obsd. (ESI⁺) [(M+H)⁺]: 253.2.

### Step 5: Preparation of methyl 3-(bromomethyl)-8-chloro-4-oxo-chromene-2-carboxylate

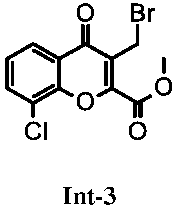

A mixture of 8-chloro-3-methyl-4-oxo-chromene-2-carboxylate (2.0 g, 7.9 mmol) and *N-*bromosuccinimide (1.8 g, 10.3 mmol), BPO (0.6 g, 2.4 mmol) in CCl₄ (10 mL) was stirred at 100 °C for 15 hours under N₂. And then, the resulting mixture was concentrated *in vacuo.* The residue was purified by silica-gel chromatography (elution with EA: PE = 10%) to afford methyl 3-(bromomethyl)-8-chloro-4-oxo-chromene-2-carboxylate (2.5 g, 86%) as a yellow solid. MS obsd. (ESI⁺) [(M+H)⁺]: 331.1.

### Intermediate 4: methyl 3-methylsulfonyloxycyclobutanecarboxylate

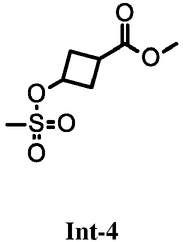

To a solution of methyl 3-hydroxycyclobutanecarboxylate (1.0 g, 7.7 mmol) and TEA (1.2 g, 11.5 mmol) in dichloromethane (10 mL) was added methanesulfonyl chloride (1.2 g, 10.0 mmol) at 0 °C and the mixture was then stirred at rt overnight. The resulting mixture was then diluted with dichloromethane (50 mL) and washed with water (20 mL) twice, saturated NaHCO₃ (20 mL) twice, brine (20 mL). The organic layer was dried over anhydrous Na₂SO₄ and concentrated *in vacuo* to give the crude methyl 3-methylsulfonyloxycyclobutanecarboxylate (1.6 g, 100%) as a colorless oil. MS obsd. (ESI⁺) [(M+H)⁺]: 209.2.

### Example 1

### tert-butyl 9-chloro-5-oxo-3,4-dihydro-1H-chromeno[2,3-c]pyridine-2(SH)-carboxylate

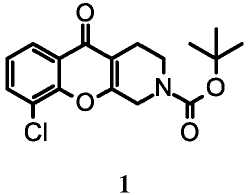

A mixture of BOC anhydride (0.93 g, 4.2 mmol) and 9-chloro-1,2,3,4-tetrahydrochromeno[2,3-c]pyridin-5-one; hydrochloride **(Int-1,** 1.0 g, 4.2 mmol) and TEA (0.89 mL, 6.4 mmol) in MeOH (10 mL) was stirred at 20 °C for 2 hours. The mixture was then concentrated *in vacuo.* The residue was purified by silica-gel chromatography (elution with EA : PE = 5%) to give *tert*-butyl 9-chloro-5-oxo-3,4-dihydro-1H-chromeno[2,3-c]pyridine-2(5H)-carboxylate (1.2g, 76.4% yield) as a white solid. ¹H NMR (CD₃OD, 400MHz): δ ppm 7.98 (d, *J*=7.2 Hz,1H), 7.81 (dd, *J*=7.6, 1.2 Hz, 1H), 7.38 (t, *J*=7.6 Hz, 1H), 4.49 (s, 2H), 3.37 (t, *J*= 4.8 Hz, 2H), 2.58 (t, *J*=5.6Hz, 2H),1.52 (s, 9H). MS obsd. (ESI⁺) [(M+H)⁺]: 336.1.

### Example 2

### 2-benzyl-9-chloro-3,4-dihydro-1H-chromeno[2,3-c]pyridin-5(2H)-one

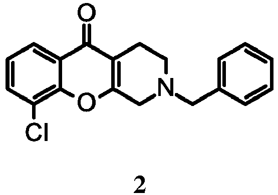

A mixture of 9-chloro-1,2,3,4-tetrahydrochromeno[2,3-c]pyridin-5-one; hydrochloride **(Int-1,** 70 mg, 0.26 mmol), bromomethylbenzene (70 mg, 0.39 mmol) and K₂CO₃ (71 mg, 0.52 mmol) in DMF (30 mL) was stirred at rt for 10 hours. And then, the resulting mixture was concentrated in *in vacuo* and the residue was purified by prep-HPLC to afford 2-benzyl-9-chloro-3,4-dihydro-1H-chromeno[2,3-c]pyridin-5(2H)-one (50 mg, 60%) as a white solid. ¹H NMR (CD₃OD, 400MHz): δ ppm 8.04 (d, *J*=6.8 Hz, 1H), 7.81 (d, *J*=9.2 HZ, 1H), 7.36-7.42 (m, 6H), 3.78 (s, 2H), 3.56 (s, 2H), 2.81 (t, *J*= 6.0 Hz, 2H), 2.62 (t, *J*=6.0 Hz, 2H). MS obsd. (ESI⁺) [(M+H)⁺]: 326.0.

### Example 3

### methyl 3-((9-chloro-5-oxo-3,4-dihydro-1H-chromeno[2,3-c]pyridin-2(SH)-yl)methyl)benzoate

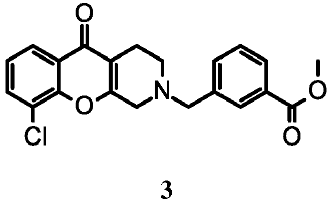

Example **3** was prepared in analogy to the procedure described for the preparation of example **2** by using methyl 3-(bromomethyl)benzoate as the starting material instead of bromomethylbenzene.

**Example** 3: ¹H NMR (CD₃OD, 400MHz): δ ppm 8.30 (s, 1H), 8.19(d, *J*=8.0Hz,1H), 8.10 (d, *J*=8.0 Hz, 1H), 7.92-7.88 (m,2H), 7.70-7.65 (m, 1H), 7.50-7.48 (m, 1H),4.62 (s, 2H), 4.38 (s, 2H), 3.95 (s, 3H), 3.34-3.32 (m, 2H), 2.94-2.91 (m, 2H). MS obsd. (ESI⁺) [(M+H)⁺]: 384.1.

### Example 4

### 9-chloro-2-(2-fluorobenzoyl)-3,4-dihydro-1H-chromeno[2,3-c]pyridin-5-one

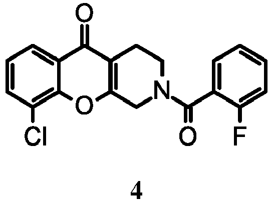

A mixture of 9-chloro-1,2,3,4-tetrahydrochromeno[2,3-c]pyridin-5-one; hydrochloride **(Int-1,** 70 mg, 0.26 mmol), 2-fluorobenzoic acid (36 mg, 0.26 mmol), HATU (114 mg, 0.30 mmol) and DIEA (66.4 mg, 0.52 mmol) in DCM (30 mL) was stirred at rt for 10 hours. And then, the resulting mixture was concentrated *in vacuo* and the residue was purified by prep-HPLC to afford 9-chloro-2-(2-fluorobenzoyl)-3,4-dihydro-1H-chromeno[2,3-c]pyridin-5(2H)-one (30mg, 33 %) as a yellow solid. ¹H NMR (DMSO-*d₆*, 400MHz): δ ppm 8.04-7.98 (m, 2H), 7.61-7.54 (m, 1H), 7.50 (t, *J*=7.9 Hz, 2H), 7.41-7.34 (m, 2H), 4.80 (s, 2H), 3.51 (t, *J*=5.7 Hz, 2H), 2.54 (t, *J*=5.7 Hz, 2H). MS obsd. (ESI⁺) [(M+H)⁺]: 358.1.

### Example 5

### 9-chloro-2-[(4-methoxyphenyl)methyl]-3,4-dihydrochromeno[2,3-c]pyridine-1,5-dione

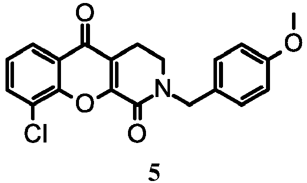

The preparation of **Example 5** was described at the preparative example **Int-2a** .

**Example 5:** ¹H NMR (DMSO-*d₆*, 400MHz): δ ppm 8.02 (ddd, *J*=1.6, 8.0, 9.5 Hz, 2H), 7.50 (t, *J*=7.95 Hz, 1H), 7.34-7.23 (m, 2H), 6.97-6.87 (m, 2H), 4.62 (s, 2H), 3.74 (s, 3H), 3.52 (t, *J*=7.03 Hz, 2H), 2.76 (t, *J*=7.03 Hz, 2H). MS obsd. (ESI⁺) [(M+H)⁺]: 370.1.

### Example 6

### 9-chloro-2-(isoquinoline-1-carbonyl)-3,4-dihydro-1H-chromeno[2,3-c]pyridin-5(2H)-one

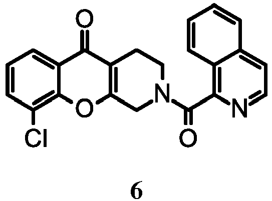

**Example 6** was prepared in analogy to the procedure described for the preparation of example **4** by using isoquinoline-1-carboxylic acid as the starting material instead of 2-fluorobenzoic acid.

**Example 6:** ¹H NMR (DMSO-*d₆*, 400MHz): δ ppm 8.61-8.53 (m, 1H), 8.12-8.06 (m, 1H), 8.02-7.82 (m, 5H), 7.77-7.67 (m, 1H), 7.54-7.41(m, 1H), 4.96 (s, 1.4 H), 4.40 (s, 0.6 H), 4.07 (t, *J*=5.8 Hz, 0.6 H), 3.39 (t, *J*=5.8 Hz, 1.4H), 2.71 (t, *J*=5.7 Hz, 0.6H), 2.43 (t, *J*=5.8 Hz, 1.4H). MS obsd. (ESI⁺) [(M+H)⁺]: 391.1.

### Example 7

### 9-chloro-N-(2-fluorophenyl)-5-oxo-3,4-dihydro-1H-chromeno[2,3-c]pyridine-2(5H)-carboxamide

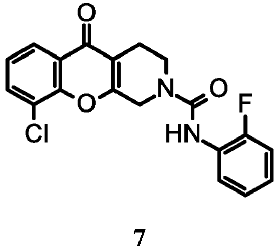

### Step 1: Preparation of N-(2-fluorophenyl)carbamoyl chloride

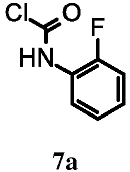

To a mixture of 2-fluoroaniline (28.6 mg, 0.26 mmol) and triphosgene (30.6 mg, 0.10 mmol) in DCM (5 mL) was added DIEA (332 mg, 2.6 mmol) dropwise at 0 °C and the mixture was stirred at 0 °C for 10 minutes. The crude mixture was used in the next step directly.

### Step 2: Preparation of 9-chloro-N-(2-fluorophenyl)-5-oxo-3,4-dihydro-1H-chromeno[2,3-c]pyridine-2(5H)-carboxamide

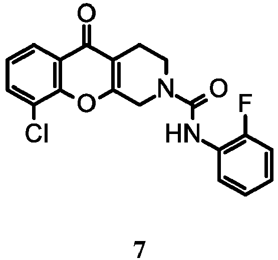

And then, 9-chloro-1,2,3,4-tetrahydrochromeno[2,3-c]pyridin-5-one; hydrochloride **(Int-1,** 70 mg, 0.26 mmol) was added to the above mixture and was stirred at rt for 1 hour. Then, the mixture was quenched with water (15 mL), extracted with DCM (10 mL) three times. The combined organic layer was dried over anhydrous Na₂SO₄ and concentrated *in vacuo.* The residue was purified by prep-HPLC to afford 9-chloro-*N*-(2-fluorophenyl)-5-oxo-3,4-dihydro-1H-chromeno[2,3-c]pyridine-2(5H)-carboxamide (20 mg, 21 %) as a yellow solid. ¹H NMR (DMSO-*d₆*, 400MHz): δ ppm 8.66 (s, 1H), 8.01 (ddd, *J*=1.5, 7.9, 15.4 Hz, 2H), 7.49 (t, *J*=7.89 Hz, 1H), 7.45-7.40 (m, 1H), 7.24-7.17 (m, 1H), 7.16-7.10 (m, 2H), 4.64 (s, 2H), 3.73 (t, *J*=5.69 Hz, 2H), 2.58 (t, *J*=5.50 Hz, 2H). MS obsd. (ESI⁺) [(M+H)⁺]: 373.2.

### Example 8

### methyl 3-(9-chloro-1,5-dioxo-3,4-dihydro-1H-chromeno[2,3-c]pyridin-2(SH)-yl)benzoate

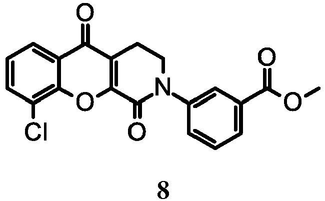

A mixture of 9-chloro-3,4-dihydro-2H-chromeno[2,3-c]pyridine-1,5-dione **(Int-2,** 100 mg, 0.41 mmol), methyl 3-iodobenzoate (210 mg, 0.80 mmol), CuI (38.2 mg, 0.20 mmol) and CS₂CO₃ (521 mg, 1.6 mmol) in DMF (5 mL) was heated at 125 °C for 1 hour in microwave reactor. And then, the mixture was poured into water (20 mL), extracted with DCM (10 mL) three times. The combined organic layer was dried over anhydrous Na₂SO₄, concentrated *in vacuo.* The residue was purified by prep-HPLC to afford methyl 3-(9-chloro-1,5-dioxo-3,4-dihydro-1H-chromeno[2,3-c]pyridin-2(5H)-yl)benzoate (20 mg, 13 % yield) as a yellow solid. ¹H NMR (DMSO-*d₆*, 400MHz): δ ppm 8.12-8.05 (m, 3H), 7.89 (d, *J*=8.0 Hz, 1H), 7.75 (d, *J*=8.0 Hz, 1H), 7.62 (t, *J*=8.0 Hz, 1H), 7.55 (t, *J*=8.0 Hz, 1H), 4.05 (t, *J*=6.9 Hz, 2H), 3.89 (s, 3H), 2.97 (t, *J*=6.9 Hz, 2H). MS obsd. (ESI⁺) [(M+H)⁺]: 384.2.

### Example 9

### 9-chloro-2-(4-methoxyphenyl)-3,4-dihydro-1H-chromeno[2,3-c]pyridine-1,5(2H)-dione

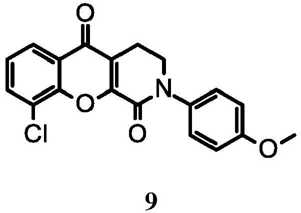

**Example 9** was prepared in analogy to the procedure described for the preparation of example **8** by using 1-iodo-4-methoxy-benzene as the starting material instead of methyl 3-iodobenzoate.

**Example 9:** ¹H NMR (DMSO-*d₆*, 400MHz): δ ppm 8.06 (d, *J*=7.95 Hz, 2H), 7.53 (d, *J*=8.0 Hz, 1H), 7.36 (d, *J*=8.0 Hz, 2H), 7.01 (d, *J*=8.0 Hz, 2H), 3.94 (t, *J*=6.9 Hz, 2H), 3.79 (s, 3H), 2.94 (t, *J*=6.9 Hz, 2H). MS obsd. (ESI⁺) [(M+H)⁺]: 356.2.

### Example 10

### methyl 4-(9-chloro-1,5-dioxo-3,4-dihydro-1H-chromeno[2,3-c]pyridin-2(SH)-yl)benzoate

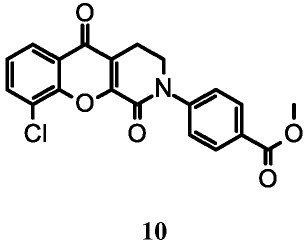

**Example 10** was prepared in analogy to the procedure described for the preparation of example **8** by using methyl 4-iodobenzoate as the starting material instead of methyl 3-iodobenzoate.

**Example 10:** ¹H NMR (DMSO-*d₆*, 400MHz): δ ppm 8.10-8.00 (m, 4H), 7.62 (t, *J*=8.0 Hz, 2H), 7.54 (t, *J*=8.0 Hz, 1H), 4.08 (t, *J*=6.9 Hz, 2H), 3.88 (s, 3H), 2.96 (t, *J*=6.9 Hz, 2H). MS obsd. (ESI⁺) [(M+H)⁺]: 384.2.

### Example 11

### 3-(4-(9-chloro-1,5-dioxo-3,4-dihydro-1H-chromeno[2,3-c]pyridin-2(SH)-yl)phenoxy)cyclobutanecarboxylic acid

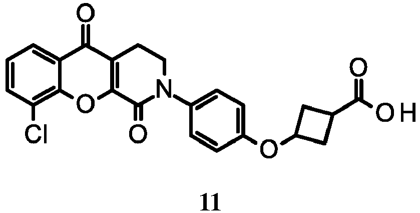

### Step 1: Preparation of 9-chloro-2-(4-hydroxyphenyl)-3,4-dihydrochromeno[2,3-c]pyridine-1,5-dione

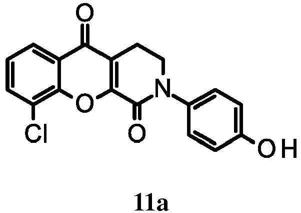

A mixture of 9-chloro-2-(4-methoxyphenyl)-3,4-dihydro-1H-chromeno[2,3-c]pyridine-1,5(2H)-dione **(Example 9,** 270 mg, 0.76 mmol) and BBr₃ (1 M in DCM, 38 mmol) in DCM (5 mL) was stirred at rt for 1 hour. And then, solvent was poured into ice-water (10 mL), extracted with DCM (10 mL) three times. The combined organic layer was dried over anhydrous Na₂SO₄ and concentrated *in vacuo* to give crude product (259mg, 100%), which was used in the next step directly. MS obsd. (ESI⁺) [(M+H)⁺]: 342.1.

### Step 2: Preparation of methyl 3-[4-(9-chloro-1,5-dioxo-3,4-dihydrochromeno[2,3-c]pyridin-2-yl)phenoxy]cyclobutanecarboxylate

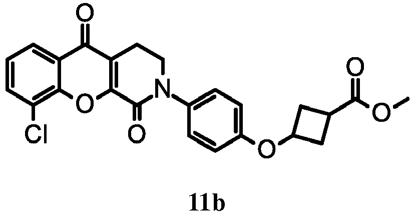

A mixture of 9-chloro-2-(4-hydroxyphenyl)-3,4-dihydrochromeno[2,3-c]pyridine-1,5-dione (80 mg, 0.23 mmol), methyl 3-methylsulfonyloxycyclobutanecarboxylate **(Int-4,** 49 mg, 0.23 mmol) and K₂CO₃ (380 mg, 1.17 mmol) in DMF (5 mL) was stirred at 70 °C for 14 hours. And then, the mixture was quenched with water (10 mL), extracted with DCM (10 mL) three times. The combined organic layer was dried over anhydrous Na₂SO₄ and concentrated *in vacuo* to give the crude methyl 3-[4-(9-chloro-1,5-dioxo-3,4-dihydrochromeno[2,3-c]pyridin-2-yl)phenoxy]cyclobutanecarboxylate (70mg, 66 %), which was used in the next step directly. MS obsd. (ESI⁺) [(M+H)⁺]: 454.2.

### Step 3: Preparation of 3-(4-(9-chloro-1,5-dioxo-3,4-dihydro-1H-chromeno[2,3-c]pyridin-2(5H)-yl)phenoxy)cyclobutanecarboxylic acid

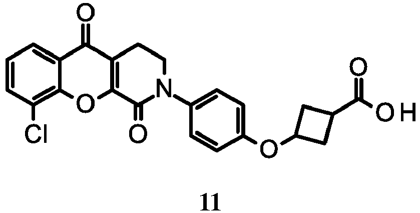

A mixture of methyl 3-[4-(9-chloro-1,5-dioxo-3,4-dihydrochromeno[2,3-c]pyridin-2-yl)phenoxy]cyclobutanecarboxylate (80mg, 0.18 mmol) in hydrochloric acid aqueous (37%wt, 10 mL) and THF (10 mL) was heated at 50 °C for 4 hours. The mixture was extracted with EA (20 mL) three times. The combined organic layer was dried over anhydrous Na₂SO₄ and concentrated *in vacuo.* The residue was purified by prep-HPLC to afford desired 3-(4-(9-chloro-1,5-dioxo-3,4-dihydro-1H-chromeno[2,3-c]pyridin-2(5H)-yl)phenoxy)cyclobutanecarboxylic acid (5mg, 6 %). ¹H NMR (DMSO-*d₆*, 400MHz): δ ppm 12.4 (brs, 1H), 8.06 (d, *J*=7.95 Hz, 2H), 7.54 (t, *J*=7.89 Hz, 1H), 7.33 (d, *J*=8.93 Hz, 2H), 6.89 (d, *J*=9.05 Hz, 2H), 4.85 (quin, *J*=6.48 Hz, 1H), 3.93 (t, *J*=6.85 Hz, 2H), 3.14-3.01 (m, 1H), 2.93 (t, *J*=6.85 Hz, 2H), 2.71-2.61 (m, 2H), 2.39-2.28 (m, 2H). MS obsd. (ESI⁺) [(M+H)⁺]: 440.2.

### Example 12

### 9-chloro-2-(4-chlorophenyl)-3,4-dihydro-1H-chromeno[2,3-c]pyridine-1,5(2H)-dione

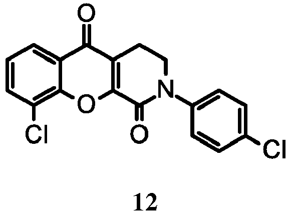

**Example 12** was prepared in analogy to the procedure described for the preparation of example **8** by using 1-chloro-4-iodobenzene as the starting material instead of methyl 3-iodobenzoate.

**Example 12:** ¹H NMR (DMSO-*d₆*, 400MHz): δ ppm 8.05 (d, *J*=7.95 Hz, 2H), 7.59-7.44 (m, 5H), 4.00 (t, *J*=6.79 Hz, 2H), 2.95 (t, *J*=6.79 Hz, 2H). MS obsd. (ESI⁺) [(M+H)⁺]: 360.1.

### Example 13

### methyl 6-(9-chloro-5-oxo-3,4-dihydro-1H-chromeno[2,3-c]pyridin-2(SH)-yl)nicotinate

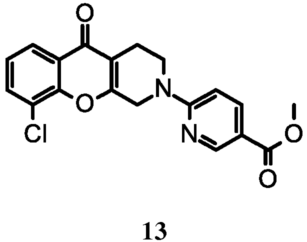

A mixture of 9-chloro-1,2,3,4-tetrahydrochromeno[2,3-c]pyridin-5-one; hydrochloride **(Int-1,** 9 mg, 0.038 mmol), methyl 6-fluoronicotinate (8.89 mg, 0.057 mmol) and DIEA (20 mg, 0.16 mmol) in DMF (2 mL) was heated at 110°C for 30 minutes in microwave reactor. And then, solvent was concentrated *in vacuo.* The residue was purified by prep-HPLC to afford methyl 6-(9-chloro-5-oxo-3,4-dihydro-1H-chromeno[2,3-c]pyridin-2(5H)-yl)nicotinate (10 mg, 70%) as a yellow solid. ¹H NMR (DMSO-*d₆*, 400MHz): δ ppm 8.72 (d, *J*=2.20 Hz, 1H), 8.14-7.92 (m, 3H), 7.49 (t, *J*=7.89 Hz, 1H), 7.11 (d, *J*=9.05 Hz, 1H), 4.82 (s, 2H), 3.97 (t, *J*=5.69 Hz, 2H), 3.81 (s, 3H), 2.63 (t, *J*=5.56 Hz, 2H). MS obsd. (ESI⁺) [(M+H)⁺]: 371.2.

### Example 14

### 9-chloro-2-(4-fluorophenyl)-3,4-dihydro-2H-chromeno[2,3-c]pyridine-1,5-dione

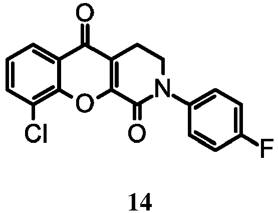

**Example 14** was prepared in analogy to the procedure described for the preparation of example **8** by using 1-fluoro-4-iodobenzene as the starting material instead of methyl 3-iodobenzoate.

**Example 14:** ¹H NMR (DMSO-*d₆*, 400MHz): δ ppm 8.06 (d, *J*=7.82 Hz, 2H), 7.57-7.45 (m, 3H), 7.37-7.25 (m, 2H), 3.98 (t, *J*=6.85 Hz, 2H), 2.95 (t, *J*=6.79 Hz, 2H). MS obsd. (ESI⁺) [(M+H)⁺]: 344.2.

### Example 15

### 2-(4-bromophenyl)-9-chloro-3,4-dihydro-2H-chromeno[2,3-c]pyridine-1,5-dione

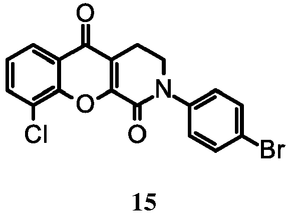

**Example 15** was prepared in analogy to the procedure described for the preparation of example **8** by using 1-bromo-4-iodobenzene as the starting material instead of methyl 3-iodobenzoate.

**Example 15:** ¹H NMR (DMSO-*d₆*, 400MHz): δ ppm 8.06 (d, *J*=7.95 Hz, 2H), 7.69-7.69 (m, 2H), 7.54 (t, *J*=7.95 Hz, 1H), 7.46-7.39 (m, 2H), 4.00 (t, *J*=6.79 Hz, 2H), 2.95 (t, *J*=6.79 Hz, 2H). MS obsd. (ESI⁺) [(M+H)⁺]: 404.2.

### Example 16

### 9-chloro-2-(6-chloropyridin-3-yl)-3,4-dihydro-2H-chromeno[2,3-c]pyridine-1,5-dione

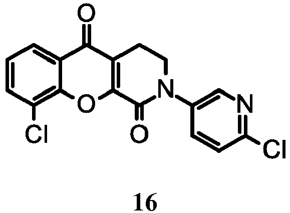

**Example 16** was prepared in analogy to the procedure described for the preparation of example **8** by using 5-bromo-2-chloro-pyridine as the starting material instead of methyl 3-iodobenzoate.

**Example 16:** ¹H NMR (DMSO-*d₆*, 400MHz): δ ppm 8.57 (d, *J*=2.69 Hz, 1H), 8.04-8.09 (m, 2H), 7.99 (dd, *J*=2.81, 8.56 Hz, 1H), 7.65 (d, *J*=8.56 Hz, 1H), 7.55 (t, *J*=7.95 Hz, 1H), 4.06 (t, *J*=6.79 Hz, 2H), 2.97 (t, *J*=6.79 Hz, 2H). MS obsd. (ESI⁺) [(M+H)⁺]: 361.1.

### Example 17

### 9-chloro-2-(4-chloro-2-methoxyphenyl)-3,4-dihydro-2H-chromeno[2,3-c]pyridine-1,5-dione

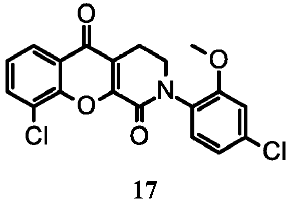

**Example 17** was prepared in analogy to the procedure described for the preparation of example **8** by using 4-chloro-1-iodo-2-methoxy-benzene as the starting material instead of methyl 3-iodobenzoate.

**Example 17:** ¹H NMR (DMSO-*d₆*, 400MHz): δ ppm 8.06 (d, *J*=7.95 Hz, 2H), 7.54 (t, *J*=7.89 Hz, 1H), 7.38 (d, *J*=8.31 Hz, 1H), 7.27 (d, *J*=2.20 Hz, 1H), 7.11 (dd, *J*=2.20, 8.31 Hz, 1H), 3.85 (s, 3H), 3.82-3.76 (m, 2H), 2.98-2.88 (m, 2H). MS obsd. (ESI⁺) [(M+H)⁺]: 390.1.

### Example 18

### 9-chloro-2-(4-fluoro-3-(trifluoromethyl)phenyl)-3,4-dihydro-2H-chromeno[2,3-c]pyridine-1,5-dione

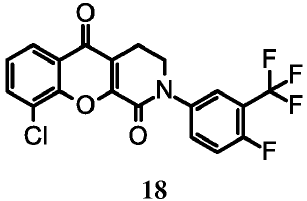

**Example 18** was prepared in analogy to the procedure described for the preparation of example **8** by using 1-fluoro-4-iodo-2-(trifluoromethyl)benzene as the starting material instead of methyl 3-iodobenzoate.

**Example 18:** ¹H NMR (DMSO-*d₆*, 400MHz): δ ppm 8.09-8.05 (m, 2H), 7.92 (dd, *J*=2.45, 6.48 Hz, 1H), 7.82-7.87 (m, 1H), 7.65 (t, *J*=9.78 Hz, 1H), 7.55 (t, *J*=7.95 Hz, 1H), 4.04 (t, *J*=6.79 Hz, 2H), 2.97 (t, *J*=6.79 Hz, 2H). MS obsd. (ESI⁺) [(M+H)⁺]: 412.1.

### Example 19

### 9-chloro-2-phenyl-3,4-dihydro-2H-chromeno[2,3-c]pyridine-1,5-dione

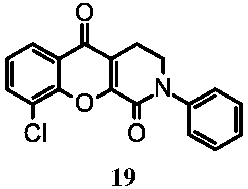

**Example 19** was prepared in analogy to the procedure described for the preparation of example **8** by using iodobenzene as the starting material instead of methyl 3-iodobenzoate.

**Example 19:** ¹H NMR (DMSO-*d₆*, 400MHz): δ ppm 8.06 (d, *J*=7.95 Hz, 2H), 7.54 (t, *J*=7.95 Hz, 1H), 7.50-7.46 (m, 4H), 7.35-7.29 (m, 1H), 4.01 (t, *J*=6.79 Hz, 2H), 2.95 (t, *J*=6.79 Hz, 2H). MS obsd. (ESI⁺) [(M+H)⁺]: 326.1.

### Example 20

### 9-chloro-2-(4-(trifluoromethyl)phenyl)-3,4-dihydro-2H-chromeno[2,3-c]pyridine-1,5-dione

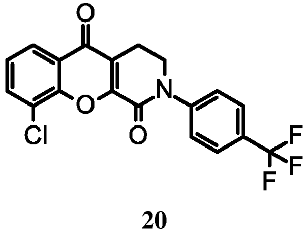

**Example 20** was prepared in analogy to the procedure described for the preparation of example **8** by using 1-iodo-4-(trifluoromethyl)benzene as the starting material instead of methyl 3-iodobenzoate.

**Example 20:** ¹H NMR (DMSO-*d₆*, 400MHz): δ ppm 8.09-8.04 (m, 2H), 7.84 (d, *J*=8.68 Hz, 2H), 7.70 (d, *J*=8.44 Hz, 2H), 7.55 (t, *J*=7.95 Hz, 1H), 4.08 (t, *J*=6.72 Hz, 2H), 2.97 (t, *J*=6.79 Hz, 2H). MS obsd. (ESI⁺) [(M+H)⁺]: 394.1.

### Example 21

### 9-chloro-2-(3-(trifluoromethoxy)phenyl)-3,4-dihydro-2H-chromeno[2,3-c]pyridine-1,5-dione

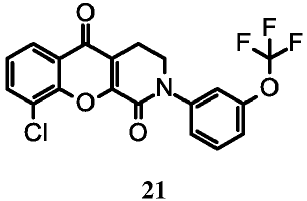

**Example 21** was prepared in analogy to the procedure described for the preparation of example **8** by using 1-iodo-3-(trifluoromethoxy)benzene as the starting material instead of methyl 3-iodobenzoate.

**Example 21:** ¹H NMR (DMSO-*d₆*, 400MHz): δ ppm 8.07 (d, *J*=7.95 Hz, 2H), 7.84 (d, *J*=6.85 Hz, 1H), 7.60 (d, *J*=5.01 Hz, 1H), 7.33 (d, *J*=7.21 Hz, 1H), 6.61 (s, 1H), 6.50 (d, *J*=7.09 Hz, 1H), 4.04 (t, *J*=6.79 Hz, 2H), 2.96 (t, *J*=6.85 Hz, 2H). MS obsd. (ESI⁺) [(M+H)⁺]: 410.1.

### Example 22

### 9-chloro-2-(4-chloro-3-(trifluoromethyl)phenyl)-3,4-dihydro-2H-chromeno[2,3-c]pyridine-1,5-dione

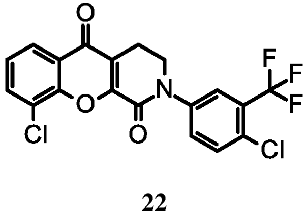

**Example 22** was prepared in analogy to the procedure described for the preparation of example **8** by using 1-chloro-4-iodo-2-(trifluoromethyl)benzene as the starting material instead of methyl 3-iodobenzoate.

**Example 22:** ¹H NMR (DMSO-*d₆*, 400MHz): δ ppm 8.09-8.04 (m, 2H), 8.00 (d, *J*=2.45 Hz, 1H), 7.87-7.83 (m, 1H), 7.81-7.76 (m, 1H), 7.55 (t, *J*=7.89 Hz, 1H), 4.06 (t, *J*=6.79 Hz, 2H), 2.97 (t, *J*=6.79 Hz, 2H). MS obsd. (ESI⁺) [(M+H)⁺]: 428.1.

### Example 23

### 9-chloro-2-(2-(trifluoromethoxy)phenyl)-3,4-dihydro-2H-chromeno[2,3-c]pyridine-1,5-dione

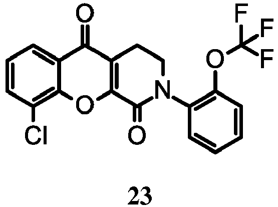

**Example 23** was prepared in analogy to the procedure described for the preparation of example **8** by using 1-iodo-2-(trifluoromethoxy)benzene as the starting material instead of methyl 3-iodobenzoate.

**Example 23:** ¹H NMR (DMSO-*d₆*, 400MHz): δ ppm 8.12 (dd, *J*=1.59, 8.07 Hz, 1H), 7.95 (dd, *J*=1.53, 7.76 Hz, 1H), 7.62-7.58 (m, 1H), 7.56-7.48 (m, 4H), 3.98 (s, 2H), 3.18-3.02 (m, 2H). MS obsd. (ESI⁺) [(M+H)⁺]: 410.1.

### Example 24

### methyl 2-chloro-5-(9-chloro-1,5-dioxo-1,3,4,5-tetrahydro-2H-chromeno[2,3-c]pyridin-2-yl)benzoate

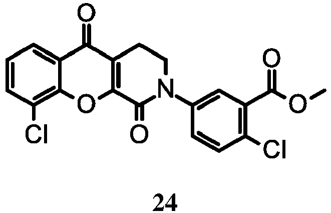

**Example 24** was prepared in analogy to the procedure described for the preparation of example **8** by using methyl 2-chloro-5-iodo-benzoate as the starting material instead of methyl 3-iodobenzoate.

**Example 24:** ¹H NMR (DMSO-*d₆*, 400MHz): δ ppm 8.07 (d, *J*=7.83 Hz, 2H), 7.97-7.90 (m, 1H), 7.71-7.67 (m, 1H), 7.70-7.67 (m, 1H), 7.55 (t, *J*=7.95 Hz, 1H), 4.07-3.99 (m, 2H), 3.89 (s, 3H), 2.96 (t, *J*=6.79 Hz, 2H). MS obsd. (ESI⁺) [(M+H)⁺]: 418.1.

### Example 25

### 3-(2-(9-chloro-1,5-dioxo-1,3,4,5-tetrahydro-2H-chromeno[2,3-c]pyridin-2-yl)phenoxy)cyclobutane-1-carboxylic acid

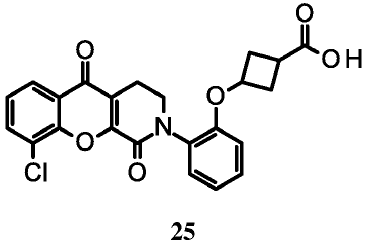

### Step1: Preparation of methyl 3-(2-iodophenoxy)cyclobutanecarboxylate

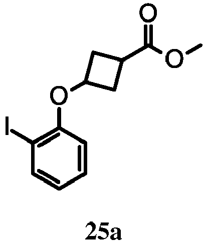

A mxiture of 2-iodophenol (1.0 g, 4.55 mmol), methyl 3-methylsulfonyloxycyclobutanecarboxylate **(Int-4,** 1.2 g, 5.5 mmol) and K₂CO₃ (1.3 g, 9.1 mmol) in DMF (20 mL) was stirred at rt for 30 minutes. Solvent was concentrated *in vacuo* and the residue was purified by silica-gel column (elution with EA : PE = 5%) to afford 3-(2-iodophenoxy)cyclobutanecarboxylate (800 mg, 53%). MS obsd. (ESI⁺) [(M+H)⁺]: 333.1.

### Step2: Preparation of methyl 3-[2-(9-chloro-1,5-dioxo-3,4-dihydrochromeno[2,3-c]pyridin-2-yl)phenoxy]cyclobutanecarboxylate

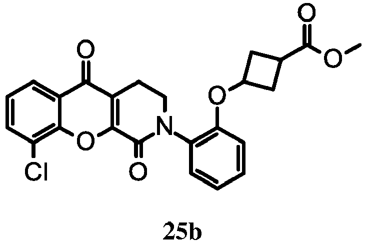

Compound **25b** was prepared in analogy to the procedure described for the preparation of example 8 by using methyl 3-(2-iodophenoxy)cyclobutanecarboxylate as the starting material instead of methyl 3-iodobenzoate. MS obsd. (ESI⁺) [(M+H)⁺]: 454.1.

### Step 3: Preparation of 3-(2-(9-chloro-1,5-dioxo-1,3,4,5-tetrahydro-2H-chromeno[2,3-c]pyridin-2-yl)phenoxy)cyclobutane-1-carboxylic acid

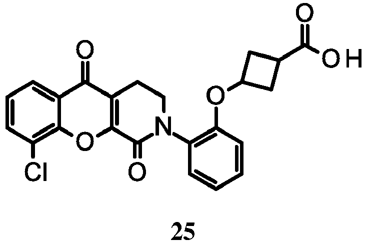

A mixture of methyl 3-[2-(9-chloro-1,5-dioxo-3,4-dihydrochromeno[2,3-c]pyridin-2-yl)phenoxy]cyclobutanecarboxylate (100 mg, 0.22 mmol) in hydrochloric acid aqueous (37%wt, 5 mL) and THF (10 mL) was heated at 60 °C for 3 hours. And then, solvent was concentrated *in vacuo* and the residue was purified by prep-HPLC to afford 3-(2-(9-chloro-1,5-dioxo-1,3,4,5-tetrahydro-2H-chromeno[2,3-c]pyridin-2-yl)phenoxy)cyclobutane-1-carboxylic acid (40 mg, 41%). ¹H NMR (DMSO-*d₆*, 400MHz): δ ppm 12.29 (br s, 1H), 8.14-8.01 (m, 2H), 7.54 (dt, *J*=1.59, 7.89 Hz, 1H), 7.28-7.40 (m, 2H), 7.00-7.06 (m, 1.5H), 6.95 (d, *J*=8.07 Hz, 0.5H), 4.90 (quin, *J*=6.39 Hz, 0.5H), 4.68-4.76 (m, 0.5H), 3.78-3.88 (m, 2H), 3.03-3.12 (m, 0.5H), 2.91-2.99 (m, 2H), 2.60-2.81 (m, 2.5H), 2.27-2.23 (m, 2H). MS obsd. (ESI⁺) [(M+H)⁺]: 440.1.

### Example 26

### 3-(5-chloro-2-(9-chloro-1,5-dioxo-1,3,4,5-tetrahydro-2H-chromeno[2,3-c]pyridin-2-yl)phenoxy)cyclobutane-1-carboxylic acid

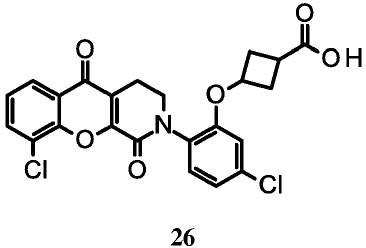

### Step1: Preparation of methyl 3-(5-chloro-2-iodo-phenoxy)cyclobutanecarboxylate

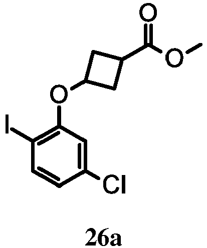

Compound **26a** was prepared in analogy to the procedure described for the preparation of Compound **25a** by using 5-chloro-2-iodophenol as the starting material instead of 2-iodophenol. MS obsd. (ESI⁺) [(M+H)⁺]: 367.1.

### Step 2: Preparation of methyl 3-[5-chloro-2-(9-chloro-1,5-dioxo-3,4-dihydrochromeno[2,3-c]pyridin-2-yl)phenoxy]cyclobutanecarboxylate

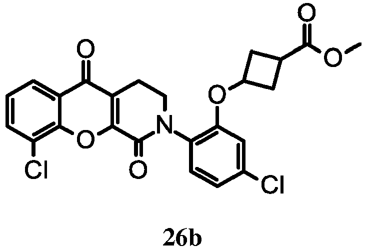

Compound **26b** was prepared in analogy to the procedure described for the preparation of example **8** by using methyl 3-(5-chloro-2-iodo-phenoxy)cyclobutanecarboxylate as the starting material instead of methyl 3-iodobenzoate. MS obsd. (ESI⁺) [(M+H)⁺]: 488.1.

### Step 3: Preparation of 3-(5-chloro-2-(9-chloro-1,5-dioxo-1,3,4,5-tetrahydro-2H-chromeno[2,3-c]pyridin-2-yl)phenoxy)cyclobutane-1-carboxylic acid

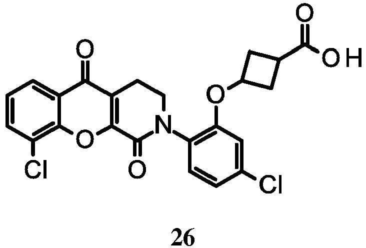

A mixture of methyl 3-[5-chloro-2-(9-chloro-1,5-dioxo-3,4-dihydrochromeno[2,3-c]pyridin-2-yl)phenoxy]cyclobutanecarboxylate (100 mg, 0.20 mmol) in hydrochloric acid aqueous (37%wt, 5 mL) and THF (10 mL) was heated at 60 °C for 3 hours. And then, solvent was concentrated *in vacuo* and the residue was purified by prep-HPLC to afford 3-(5-chloro-2-(9-chloro-1,5-dioxo-1,3,4,5-tetrahydro-2H-chromeno[2,3-c]pyridin-2-yl)phenoxy)cyclobutane-1-carboxylic acid (50 mg, 51%). ¹H NMR (DMSO-*d₆*, 400MHz): δ ppm 12.33 (br s, 1H), 8.08-8.03 (m, 2H), 7.58-7.51 (m, 1H), 7.42-7.36 (m, 1H), 7.10 (dd, *J*=2.14, 8.50 Hz, 1H), 7.01 (d, *J*=2.20 Hz, 1H), 4.96 (quin, *J*=6.20 Hz, 1H), 3.83 (t, *J*=6.72 Hz, 2H), 3.14-3.04 (m, 1H), 2.95 (br t, *J*=6.60 Hz, 2H), 2.77-2.56 (m, 2H), 2.38-2.26 (m, 2H). MS obsd. (ESI⁺) [(M+H)⁺]: 474.2.

### Example 27

### 9-chloro-2-(4-chloro-2-(trifluoromethyl)phenyl)-3,4-dihydro-2H-chromeno[2,3-c]pyridine-1,5-dione

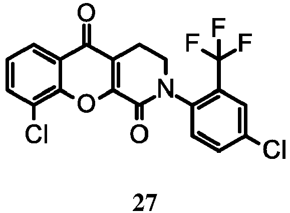

**Example 27** was prepared in analogy to the procedure described for the preparation of example **8** by using 4-chloro-1-iodo-2-(trifluoromethyl)benzene as the starting material instead of methyl 3-iodobenzoate.

**Example 27:** ¹H NMR (DMSO-*d₆*, 400MHz): δ ppm 8.07 (d, *J*=7.95 Hz, 2H), 8.01-7.95 (m, 2H), 7.77 (d, *J*=7.95 Hz, 1H), 7.55 (t, *J*=7.95 Hz, 1H), 4.10 (dt, *J*=4.89, 12.47 Hz, 1H), 3.67-3.58 (m, 1H), 3.03 (td, *J*=4.10, 16.99 Hz, 1H), 2.90-2.78 (m, 1H). MS obsd. (ESI⁺) [(M+H)⁺]: 428.1.

### Example 28

### 9-chloro-2-(3,4-dichlorophenyl)-3,4-dihydro-2H-chromeno[2,3-c]pyridine-1,5-dione

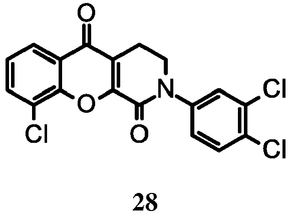

**Example 28** was prepared in analogy to the procedure described for the preparation of example **8** by using 1,2-dichloro-4-iodo-benzene as the starting material instead of methyl 3-iodobenzoate.

**Example 28:** ¹H NMR (DMSO-*d₆*, 400MHz): δ ppm 8.06 (qd, *J*=1.47, 7.93 Hz, 2H), 7.80 (d, *J*=2.45 Hz, 1H), 7.74 (d, *J*=8.68 Hz, 1H), 7.55 (t, *J*=7.89 Hz, 1H), 7.49 (dd, *J*=2.45, 8.68 Hz, 1H), 4.02 (t, *J*=6.79 Hz, 2H), 2.95 (t, *J*=6.79 Hz, 2H). MS obsd. (ESI⁺) [(M+H)⁺]: 394.1.

### Example 29

### 9-chloro-2-(4-chloro-2-fluorophenyl)-3,4-dihydro-2H-chromeno[2,3-c]pyridine-1,5-dione

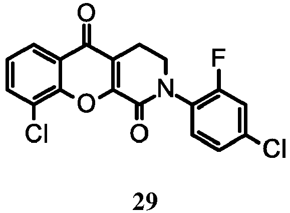

**Example 29** was prepared in analogy to the procedure described for the preparation of example **8** by using 4-chloro-2-fluoro-1-iodo-benzene as the starting material instead of methyl 3-iodobenzoate.

**Example 29:** ¹H NMR (DMSO-*d₆*, 400MHz): δ ppm 8.11-8.00 (m, 2H), 7.64 (dd, *J*=2.26, 10.21 Hz, 1H), 7.56 (td, *J*=8.24, 16.66 Hz, 2H), 7.45-7.41 (m, 1H), 3.95 (t, *J*=6.79 Hz, 2H), 2.96 (t, *J*=6.79 Hz, 2H). MS obsd. (ESI⁺) [(M+H)⁺]: 378.1.

### Example 30

### 4-(9-chloro-1,5-dioxo-1,3,4,5-tetrahydro-2H-chromeno[2,3-c]pyridin-2-yl)benzonitrile

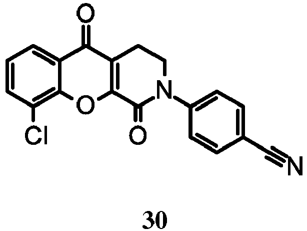

**Example 30** was prepared in analogy to the procedure described for the preparation of example **8** by using 4-iodobenzonitrile as the starting material instead of methyl 3-iodobenzoate.

**Example 30:** ¹H NMR (DMSO-*d₆*, 400MHz): δ ppm 8.08-8.06 (m, 2H), 7.95 (d, *J*=8.68 Hz, 2H), 7.68 (d, *J*=8.80 Hz, 2H), 7.55 (t, *J*=7.89 Hz, 1H), 4.08 (t, *J*=6.72 Hz, 2H), 2.96 (t, *J*=6.72 Hz, 2H). MS obsd. (ESI⁺) [(M+H)⁺]: 351.1.

### Example 31

### 9-chloro-2-(2,5-dimethylphenyl)-3,4-dihydro-2H-chromeno[2,3-c]pyridine-1,5-dione

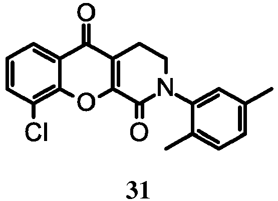

**Example 31** was prepared in analogy to the procedure described for the preparation of example **8** by using 2-iodo-1,4-dimethyl-benzene as the starting material instead of methyl 3-iodobenzoate.

**Example 31:** ¹H NMR (DMSO-*d₆*, 400MHz): δ ppm 8.06 (d, *J*=7.95 Hz, 2H), 7.54 (t, *J*=7.95 Hz, 1H), 7.23 (d, *J*=7.70 Hz, 1H), 7.14 (s, 1H), 7.11 (d, *J*=7.70 Hz, 1H), 3.99 (ddd, *J*=6.11, 9.84, 12.41 Hz, 1H), 3.73-3.67 (m, 1H), 3.04-2.90 (m, 2H), 2.30 (s, 3H), 2.18 (s, 3H). MS obsd. (ESI⁺) [(M+H)⁺]: 354.2.

### Example 32

### 9-chloro-2-(2-chloro-4-(trifluoromethoxy)phenyl)-3,4-dihydro-2H-chromeno[2,3-c]pyridine-1,5-dione

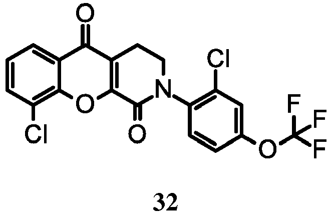

**Example 32** was prepared in analogy to the procedure described for the preparation of example **8** by using 2-chloro-1-iodo-4-(trifluoromethoxy)benzene as the starting material instead of methyl 3-iodobenzoate.

**Example 32:** ¹H NMR (DMSO-*d₆*, 400MHz): δ ppm 8.07 (d, *J*=7.95 Hz, 2H), 7.81 (q, *J*=2.98 Hz, 1H), 7.74 (d, *J*=8.80 Hz, 1H), 7.56 (d, *J*=7.83 Hz, 2H), 4.10-3.94 (m, 1H), 3.79 (td, *J*=5.79, 12.01 Hz, 1H), 3.08-2.87 (m, 2H). MS obsd. (ESI⁺) [(M+H)⁺]: 444.1.

### Example 33

### 9-chloro-2-(4-(trifluoromethoxy)phenyl)-3,4-dihydro-2H-chromeno[2,3-c]pyridine-1,5-dione

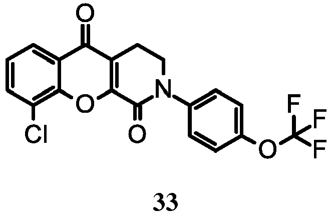

**Example 33** was prepared in analogy to the procedure described for the preparation of example **8** by using 1-iodo-4-(trifluoromethoxy)benzene as the starting material instead of methyl 3-iodobenzoate.

**Example 33:** ¹H NMR (DMSO-*d₆*, 400MHz): δ ppm 8.07 (d, *J*=7.95 Hz, 2H), 7.65-7.60 (m, 3H), 7.48 (d, *J*=8.31 Hz, 2H), 4.02 (t, *J*=6.79 Hz, 2H), 2.95 (t, *J*=6.85 Hz, 2H). MS obsd.

(ESI⁺) [(M+H)⁺]: 410.1.

### Example 34

### 9-chloro-2-(p-tolyl)-3,4-dihydro-2H-chromeno[2,3-c]pyridine-1,5-dione

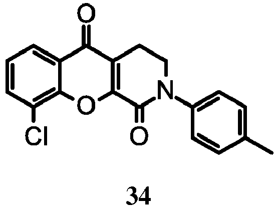

**Example 34** was prepared in analogy to the procedure described for the preparation of example **8** by using 1-iodo-4-methyl-benzene as the starting material instead of methyl 3-iodobenzoate.

**Example 34:** ¹H NMR (DMSO-*d₆*, 400MHz): δ ppm 8.05 (d, *J*=7.83 Hz, 2H), 7.54 (t, *J*=7.89 Hz, 1H), 7.37-7.30 (m, 2H), 7.30-7.20 (m, 2H), 3.97 (t, *J*=6.79 Hz, 2H), 2.94 (t, *J*=6.85 Hz, 2H), 2.34 (s, 3H). MS obsd. (ESI⁺) [(M+H)⁺]: 340.1.

### Example 35

### 9-chloro-2-(1-methyl-1H-pyrazol-4-yl)-3,4-dihydro-2H-chromeno[2,3-c]pyridine-1,5-dione

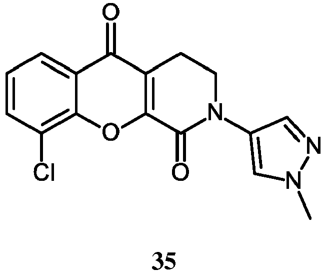

**Example 35** was prepared in analogy to the procedure described for the preparation of example **8** by using 4-iodo-1-methyl-pyrazole as the starting material instead of methyl 3-iodobenzoate.

**Example 35:** ¹H NMR (DMSO-*d₆*, 400MHz): δ ppm 8.18 (s, 1H), 8.05 (qd, *J*=1.37, 7.89 Hz, 2H), 7.78 (s, 1H), 7.53 (t, *J*=7.89 Hz, 1H), 4.01 (t, *J*=6.97 Hz, 2H), 3.86 (s, 3H), 2.93 (t, *J*=7.03 Hz, 2H). MS obsd. (ESI⁺) [(M+H)⁺]: 330.1.

### Example 36

### methyl 5-chloro-2-(9-chloro-1,5-dioxo-1,3,4,5-tetrahydro-2H-chromeno[2,3-c]pyridin-2-yl)benzoate

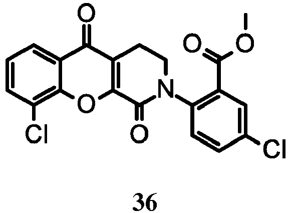

**Example 36** was prepared in analogy to the procedure described for the preparation of example **8** by using methyl 5-chloro-2-iodo-benzoate as the starting material instead of methyl 3-iodobenzoate.

**Example 36:** ¹H NMR (DMSO-*d₆*, 400MHz): δ ppm 8.06 (qd, *J*=1.43, 7.93 Hz, 2H), 7.91 (d, *J*=2.45 Hz, 1H), 7.84 (dd, *J*=2.57, 8.44 Hz, 1H), 7.60 (d, *J*=8.44 Hz, 1H), 7.55 (t, *J*=7.89 Hz, 1H), 3.96 (br s, 2H), 3.77 (s, 3H), 3.08-2.86 (m, 2H). MS obsd. (ESI⁺) [(M+H)⁺]: 418.2.

### Example 37

### 5-chloro-2-(9-chloro-1,5-dioxo-1,3,4,5-tetrahydro-2H-chromeno[2,3-c]pyridin-2-yl)benzonitrile

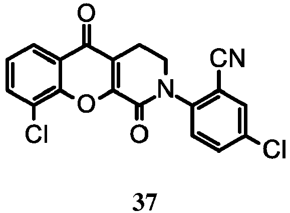

**Example 37** was prepared in analogy to the procedure described for the preparation of example **8** by using 5-chloro-2-iodo-benzonitrile as the starting material instead of methyl 3-iodobenzoate.

**Example 37:** ¹H NMR (DMSO-*d₆*, 400MHz): δ ppm 8.21 (d, *J*=2.20 Hz, 1H), 8.07 (dd, *J*=1.47, 7.95 Hz, 2H), 7.96 (dd, *J*=2.32, 8.68 Hz, 1H), 7.70 (d, *J*=8.68 Hz, 1H), 7.55 (t, *J*=7.89 Hz, 1H), 4.03 (br s, 2H), 2.99 (br t, *J*=6.48 Hz, 2H). MS obsd. (ESI⁺) [(M+H)⁺]: 385.2.

### Example 38

### 9-chloro-2-(4-chloro-2-methylphenyl)-3,4-dihydro-2H-chromeno[2,3-c]pyridine-1,5-dione

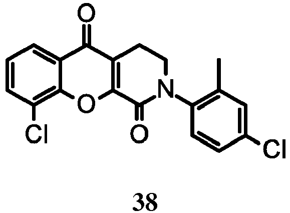

**Example 38** was prepared in analogy to the procedure described for the preparation of example **8** by using 4-chloro-1-iodo-2-methyl-benzene as the starting material instead of methyl 3-iodobenzoate.

**Example 38:** ¹H NMR (DMSO-*d₆*, 400MHz): δ ppm 8.06 (d, *J*=7.95 Hz, 2H), 7.54 (t, *J*=7.89 Hz, 1H), 7.46 (s, 1H), 7.38 (d, *J*=1.34 Hz, 2H), 4.08-3.94 (m, 1H), 3.75-3.64 (m, 1H), 3.00-2.93 (m, 2H), 2.23 (s, 3H). MS obsd. (ESI⁺) [(M+H)⁺]: 374.1.

### Example 39

### 9-chloro-2-(4-(trifluoromethyl)phenyl)-1,2,3,4-tetrahydro-5H-chromeno[2,3-c]pyridin-5-one

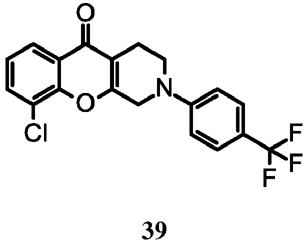

A mixture of 9-chloro-1,2,3,4-tetrahydrochromeno[2,3-c]pyridin-5-one; hydrochloride **(Int-1,** 27.2 mg, 0.1 mmol), (4-(trifluoromethyl)phenyl)boronic acid (57 mg, 0.3 mmol), Cu(OAc)₂ (36.3 mg, 200 µmol), MS 4A (30 mg, 100 µmol) and TEA (30.4 mg, 300 µmol) in DCM (2 mL) was stirred at rt for 2 hours under air. The reaction mixture was diluted with DCM (30 mL) and then filtered, the filtrate was washed with ammonia hydroxide (20 mL) twice, brine (20 mL). The organic layer was dried over anhydrous Na₂SO₄ and concentrated *in vacuo.* The residue was purified by prep-HPLC to give 9-chloro-2-(4-(trifluoromethyl)phenyl)-1,2,3,4-tetrahydro-5H-chromeno[2,3-c]pyridin-5-one (5 mg, 13%) as a yellow solid. ¹H NMR (DMSO-*d₆*, 400MHz): δ ppm 8.01 (ddd, *J*=1.53, 7.92, 14.64 Hz, 2H), 7.56 (d, *J*=8.68 Hz, 2H), 7.49 (t, *J*=7.89 Hz, 1H), 7.24 (d, *J*=8.80 Hz, 2H), 4.50 (s, 2H), 3.68 (t, *J*=5.69 Hz, 2H), 2.69-2.62 (m, 2H). MS obsd. (ESI⁺) [(M+H)⁺]: 380.1.

### Example 40

### 9-chloro-2-(4-methoxyphenyl)-1,2,3,4-tetrahydro-5H-chromeno[2,3-c]pyridin-5-one

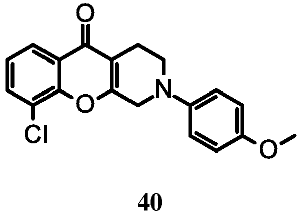

**Example 40** was prepared in analogy to the procedure described for the preparation of example **39** by using (4-methoxyphenyl)boronic acid as the starting material instead of (4-(trifluoromethyl)phenyl)boronic acid.

**Example 40:** ¹H NMR (DMSO-*d₆*, 400MHz): δ ppm 8.00 (ddd, *J*=1.6, 7.9, 16.9 Hz, 2H), 7.48 (t, *J*=7.9 Hz, 1H), 7.09-7.03 (m, 2H), 6.90-6.83 (m, 2H), 4.24 (s, 2H), 3.70 (s, 3H), 3.41 (t, *J*=5.7 Hz, 2H), 2.61 (t, *J*=5.6 Hz, 2H). MS obsd. (ESI⁺) [(M+H)⁺]: 342.1.

### Example 41

### 9-chloro-2-(3-methoxyphenyl)-1,2,3,4-tetrahydro-5H-chromeno[2,3-c]pyridin-5-one

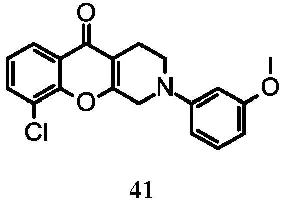

**Example 41** was prepared in analogy to the procedure described for the preparation of example **39** by using (3-methoxyphenyl)boronic acid as the starting material instead of (4-(trifluoromethyl)phenyl)boronic acid.

**Example 41:** ¹H NMR (DMSO-*d₆*, 400MHz): δ ppm 8.01 (ddd, *J*=1.6, 7.9, 16.0 Hz, 2H), 7.49 (t, *J*=7.9 Hz, 1H), 7.19-7.14 (m, 1H), 6.72-6.60 (m, 3H), 4.36 (s, 2H), 3.75 (s, 3H), 3.53 (t, *J*=5.6 Hz, 2H), 2.62 (t, *J*=5.5 Hz, 2H). MS obsd. (ESI⁺) [(M+H)⁺]: 342.1.

### Example 42

### 9-chloro-2-(4-chlorophenyl)-1,2,3,4-tetrahydro-5H-chromeno[2,3-c]pyridin-5-one

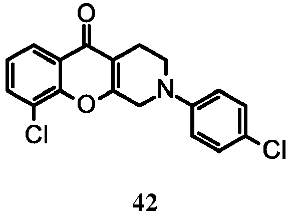

**Example 42** was prepared in analogy to the procedure described for the preparation of example **39** by using (4-chlorophenyl)boronic acid as the starting material instead of (4-(trifluoromethyl)phenyl)boronic acid.

**Example 42:** ¹H NMR (DMSO-*d₆*, 400MHz): δ ppm 8.00 (ddd, *J*=1.5, 7.9, 15.0 Hz, 2H), 7.48 (t, *J*=7.9 Hz, 1H), 7.32-7.24 (m, 2H), 7.16-7.09 (m, 2H), 4.36 (s, 2H), 3.54 (t, *J*=5.7 Hz, 2H), 2.62 (t, *J*=5.6 Hz, 2H). MS obsd. (ESI⁺) [(M+H)⁺]: 346.1.

### Example 43

### 9-chloro-2-(5-chloro-3-(trifluoromethyl)pyridin-2-yl)-3,4-dihydro-2H-chromeno[2,3-c]pyridine-1,5-dione

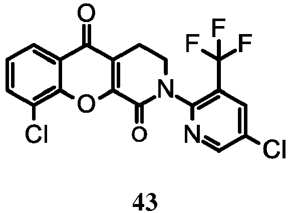

A mixture of 9-chloro-3,4-dihydro-2H-chromeno[2,3-c]pyridine-1,5-dione **(Int-2,** 37 mg, 0.15 mmol), 2-bromo-5-chloro-3-(trifluoromethyl)pyridine (78 mg, 0.3 mmol), CuI (3 mg, 0.015 mmol), *N*,*N*-dimethylglycine (3 mg, 0.03 mmol) and potassiumphosphate tribasic (64 mg, 0.3 mmol) in DMF (0.5 mL) was heated to 100 °C for 16 hours. After cooling to rt, the mixture was directly purified by prep-HPLC to afford 9-chloro-2-(5-chloro-3-(trifluoromethyl)pyridin-2-yl)-3,4-dihydro-2H-chromeno[2,3-c]pyridine-1,5-dione (6 mg, 8%) as a yellow solid. ¹H NMR (DMSO-*d₆*, 400MHz): δ ppm 9.00 (d, *J*=2.3 Hz, 1H), 8.64 (d, *J*=2.4 Hz, 1H), 8.09-8.04 (m, 2H), 7.58-7.52 (m, 1H), 4.25-3.76 (m, 2H), 3.10-2.80 (m, 2H). MS obsd. (ESI⁺) [(M+H)⁺]: 429.1.

### Example 44

### 2-(2-bromo-4-chlorophenyl)-9-chloro-3,4-dihydro-2H-chromeno[2,3-c]pyridine-1,5-dione

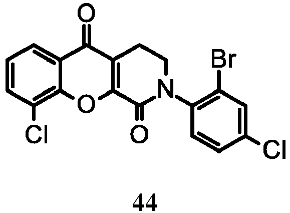

**Example 44** was prepared in analogy to the procedure described for the preparation of example **43** by using 2-bromo-4-chloro-1-iodobenzene as the starting material instead of 2-bromo-5-chloro-3-(trifluoromethyl)pyridine.

**Example 44:** ¹H NMR (DMSO-*d₆*, 400MHz): δ ppm 8.09-8.04 (m, 2H), 7.97 (d, J=1.8 Hz, 1H), 7.64-7.52 (m, 3H), 4.05-3.69 (m, 2H), 3.10-2.90 (m, 2H). MS obsd. (ESI⁺) [(M+H)⁺]: 438.1.

### Example 45

### 9-chloro-2-(5-chloro-3-((4-methoxybenzyl)oxy)pyridin-2-yl)-3,4-dihydro-2H-chromeno[2,3-c]pyridine-1,5-dione

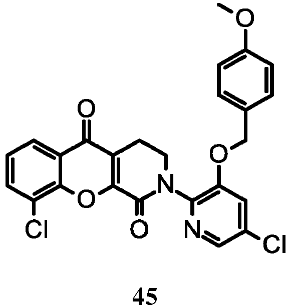

**Example 45** was prepared in analogy to the procedure described for the preparation of example **43** by using 2-bromo-5-chloro-3-((4-methoxybenzyl)oxy)pyridine as the starting material instead of 2-bromo-5-chloro-3-(trifluoromethyl)pyridine.

**Example 45:** ¹H NMR (DMSO-*d₆*, 400MHz): δ ppm 8.19 (d, *J*=2.1 Hz, 1H), 8.06 (ddd, *J*=1.5, 8.0, 9.7 Hz, 2H), 7.90 (d, *J*=2.1 Hz, 1H), 7.54 (t, *J*=7.9 Hz, 1H), 7.39 (d, *J*=8.7 Hz, 2H), 6.91-6.84 (m, 2H), 5.19 (s, 2H), 3.68 (s, 3H), 3.40-3.25 (m, 2H), 3.75-3.00 (m, 2H). MS obsd.

(ESI⁺) [(M+H)⁺]: 498.2.

### Example 46

### 9-chloro-2-(4-chloro-2-(methoxymethoxy)phenyl)-3,4-dihydro-2H-chromeno[2,3-c]pyridine-1,5-dione

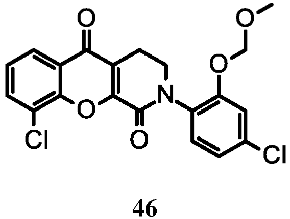

**Example 46** was prepared in analogy to the procedure described for the preparation of example 8 by using 4-chloro-1-iodo-2-(methoxymethoxy)benzene as the starting material instead of methyl 3-iodobenzoate.

**Example 46**: ¹H NMR (DMSO-d₆, 400MHz): δ ppm 8.06 (d, *J*=7.95 Hz, 2H), 7.54 (t, *J*=7.89 Hz, 1H), 7.42 (d, *J*=8.31 Hz, 1H), 7.32 (d, *J*=2.20 Hz, 1H), 7.17 (dd, *J*=2.20, 8.44 Hz, 1H), 5.29 (s, 2H), 3.83 (br t, *J*=6.66 Hz, 2H), 3.38 (s, 3H), 2.95 (t, *J*=6.54 Hz, 2H). MS obsd. (ESI⁺) [(M+H)⁺]: 420.1.

### Example 47

### 9-chloro-2-(4-chloro-2-(pyrrolidine-1-carbonyl)phenyl)-3,4-dihydro-2H-chromeno[2,3-c]pyridine-1,5-dione

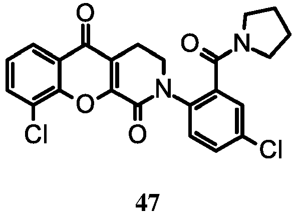

### Step 1: Preparation of 5-chloro-2-(9-chloro-1,5-dioxo-3,4-dihydrochromeno[2,3-c]pyridin-2-yl)benzoic acid

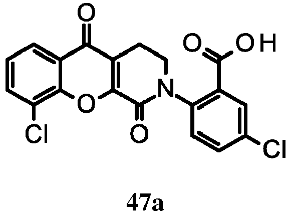

A mixture of 5-chloro-2-(9-chloro-1,5-dioxo-1,3,4,5-tetrahydro-2H-chromeno[2,3-c]pyridin-2-yl)benzoate (**Example 36**, 100 mg, 0.24 mmol) in hydrochloric acid aqueous (37%wt, 5 mL) and THF (10mL) was heated at 60 °C for 18 hours. The mixture was extracted with DCM (20 mL) three times. The combined organic layer was dried over anhydrous Na₂SO₄ and concentrated *in vacuo.* The residue was suspended in diethyl ether (5 mL) and the suspension was collected by filtration to afford 5-chloro-2-(9-chloro-1,5-dioxo-3,4-dihydrochromeno[2,3-c]pyridin-2-yl)benzoic acid (90 mg, 93%). MS obsd. (ESI⁺) [(M+H)⁺]: 404.1.

### Step 2: Preparation of 9-chloro-2-(4-chloro-2-(pyrrolidine-1-carbonyl)phenyl)-3,4-dihydro-2H-chromeno[2,3-c]pyridine-1,5-dione

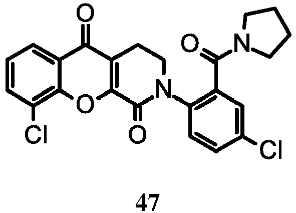

A mixture of 5-chloro-2-(9-chloro-1,5-dioxo-3,4-dihydrochromeno[2,3-c]pyridin-2-yl)benzoic acid (30 mg, 74.2 µmol), pyrrolidinyl (10.6 mg, 148 µmol), HATU (33.8 mg, 89.1 µmol,) and DIEA (19.1 mg, 148 µmol) in DCM (3 mL) was stirred at rt for 3 hours. And then, solvent was evaporated and the residue was purified by prep-HPLC to afford 9-chloro-2-(4-chloro-2-(pyrrolidine-1-carbonyl)phenyl)-3,4-dihydro-2H-chromeno[2,3-c]pyridine-1,5-dione (5 mg, 15%). ¹H NMR (DMSO-d₆, 400MHz): δ ppm 8.06 (d, *J*=7.95 Hz, 2H), 7.65-7.59 (m, 2H), 7.58-7.50 (m, 2H), 3.85 (br s, 2H), 3.34-3.32 (m, 2H), 3.28 (t, *J*=6.36 Hz, 2H), 2.88 (d, *J*=6.36 Hz, 2H), 1.87-1.73 (m, 4H). MS obsd. (ESI⁺) [(M+H)⁺]: 457.1.

### Example 48

### 9-chloro-2-(4-chloro-2-ethoxyphenyl)-3,4-dihydro-2H-chromeno[2,3-c]pyridine-1,5-dione

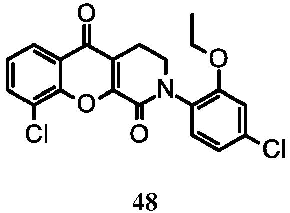

### Step 1: Preparation of 9-chloro-2-(4-chloro-2-hydroxy-phenyl)-3,4-dihydrochromeno[2,3-c]pyridine-1,5-dione

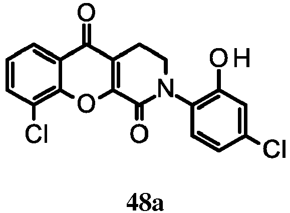

A mixture of 9-chloro-2-(4-chloro-2-(methoxymethoxy)phenyl)-3,4-dihydro-2H-chromeno[2,3-c]pyridine-1,5-dione (**Example 46**, 100 mg, 0.24 mmol) in hydrochloric acid aqueous (37%wt, 1 mL) and water (5 mL) was stirred at rt for 5 hours. And then, solvent was evaporated and the residue was purified by prep-HPLC to afford 9-chloro-2-(4-chloro-2-hydroxy-phenyl)-3,4-dihydrochromeno[2,3-c]pyridine-1,5-dione (60 mg, 67%). MS obsd. (ESI⁺) [(M+H)⁺]: 376.2.

### Step 2: Preparation of 9-chloro-2-(4-chloro-2-ethoxyphenyl)-3,4-dihydro-2H-chromeno[2,3-c]pyridine-1,5-dione

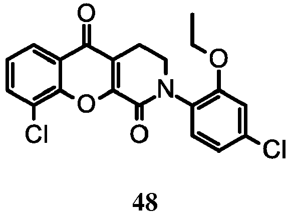

A mixture of 9-chloro-2-(4-chloro-2-hydroxy-phenyl)-3,4-dihydrochromeno[2,3-c]pyridine-1,5-dione (45mg, 0.12 mmol), iodoethane (93.3 mg, 0.60 mmol) and Cs₂CO₃ (0.36 mmol) in DMF (5 ml) was stirred at rt for 10 hours. And then, the mixture was quenched with water (10 mL), extracted with DCM (20 mL) three times. The combined organic layer was dried over anhydrous Na₂SO₄ and concentrated *in vacuo.* The residue was purified by prep-HPLC to afford 9-chloro-2-(4-chloro-2-ethoxyphenyl)-3,4-dihydro-2H-chromeno[2,3-c]pyridine- 1,5-dione (10 mg, 21%). ¹H NMR (DMSO-d₆, 400MHz): δ ppm 8.06 (d, *J*=7.83 Hz, 2H), 7.54 (t, *J*=7.95 Hz, 1H), 7.38 (d, *J*=8.31 Hz, 1H), 7.26 (d, *J*=2.20 Hz, 1H), 7.09 (dd, *J*=2.20, 8.31 Hz, 1H), 4.13 (q, *J*=6.85 Hz, 2H), 3.80 (t, *J*=6.79 Hz, 2H), 2.93 (br t, *J*=6.54 Hz, 2H), 1.31 (t, *J*=6.97 Hz, 3H). MS obsd. (ESI⁺) [(M+H)⁺]: 404.2.

### Example 49

### 9-chloro-2-(3,4-dichlorophenyl)-1,2,3,4-tetrahydro-5H-chromeno[2,3-c]pyridin-5-one

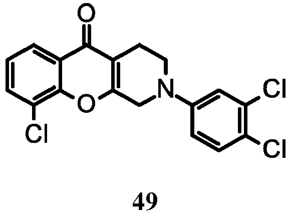

**Example 49** was prepared in analogy to the procedure described for the preparation of example **39** by using (3,4-dichlorophenyl)boronic acid as the starting material instead of (4-(trifluoromethyl)phenyl)boronic acid.

**Example 49**: ¹H NMR (DMSO-*d₆*, 400MHz): δ ppm 8.01 (ddd, *J*=1.53, 7.92, 14.52 Hz, 2H), 7.56-7.42 (m, 2H), 7.35 (d, *J*=2.93 Hz, 1H), 7.11 (dd, *J*=2.93, 9.05 Hz, 1H), 4.43 (s, 2H), 3.58 (t, *J*=5.69 Hz, 2H), 2.63 (br t, *J*=5.56 Hz, 2H). MS obsd. (ESI⁺) [(M+H)⁺]: 380.1.

### Example 50

### 9-chloro-2-(4-chloro-2-(methoxymethyl)phenyl)-3,4-dihydro-2H-chromeno[2,3-c]pyridine-1,5-dione

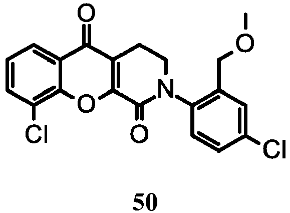

**Example 50** was prepared in analogy to the procedure described for the preparation of example 8 by using 4-chloro-1-iodo-2-(methoxymethyl)benzene as the starting material instead of methyl 3-iodobenzoate.

**Example 50:** ¹H NMR (DMSO-*d₆*, 400MHz): δ ppm 8.02 (d, *J*=7.95 Hz, 2H), 7.54 (t, *J*=7.89 Hz, 1H), 7.28 (d, *J*=8.31 Hz, 2H), 6.94 (d, *J*=2.20 Hz, 1H), 4.94 (s, 2H), 4.13 (q, *J*=6.85 Hz, 2H), 3.75 (s, 3H), 2.93 (t, *J*=6.54 Hz, 2H). MS obsd. (ESI⁺) [(M+H)⁺]: 404.2.

### Example 51

### 5-chloro-2-(4-chlorophenyl)-1H-chromeno[2,3-c]pyrrole-3,9-dione

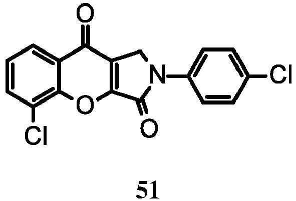

A mixture of 4-chloroaniline (69.3 mg, 0.54 mmol) and methyl 3-(bromomethyl)-8-chloro-4-oxo-chromene-2-carboxylate (**Int-3**, 150 mg, 0.45 mmol) in ethanol (8 mL) was stirred at 80 °C for 2 hours. The mixture was concentrated *in vacuo* and the residue was purified by Prep-HPLC to give 5-chloro-2-(4-chlorophenyl)-1H-chromeno[2,3-c]pyrrole-3,9-dione (11 mg, 7%) as a white solid. ¹H NMR (DMSO-*d₆*, 400MHz): δ ppm 8.23-8.07 (m, 2H), 8.03-7.91 (m, 1H), 7.97 (d, *J*=9.0 Hz, 1H), 7.65-7.50 (m, 3H), 4.95 (s, 2H). MS obsd. (ESI⁺) [(M+H)⁺]: 345.9.

### Example 52

### 5-chloro-2-(4-methoxyphenyl)-1H-chromeno[2,3-c]pyrrole-3,9-dione

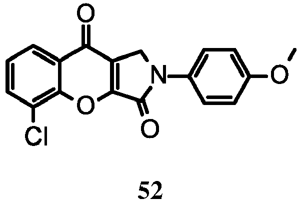

**Example 52** was prepared in analogy to the procedure described for the preparation of example 51 by using p-anisidine as the starting material instead of 4-chloroaniline.

**Example 52**: ¹H NMR (DMSO-d₆, 400MHz): δ ppm 8.13 (dd, *J*=7.9, 13.9 Hz, 2H), 7.83 (d, *J*=9.0 Hz, 2H), 7.61 (s, 1H), 7.05 (d, *J*=9.0 Hz, 2H), 4.91 (s, 2H), 3.79 (s, 3H). MS obsd. (ESI⁺) [(M+H)⁺]: 341.9.

### BIOLOGICAL EXAMPLES

### Example 53: Engineered HepDES19 primary screen assay

The assay was employed to screen for novel cccDNA inhibitors. HepDES19 is a cccDNA-producing cell line. In this cell line, HBeAg in the cell culture supernatant as surrogate marker, as HBeAg production depends on cccDNA level and activity. HepDES19 is an engineered cell line which contains a 1.1 unit length HBV genome, and pgRNA transcription from the transgene is controlled by Tetracycline (Tet). In the absence of Tet, pgRNA transcription will be induced, but HBV e antigen (HBeAg) could not be produced from this pgRNA due to very short leader sequence before the HBeAg start codon and the start codon is disrupted. Only after cccDNA is formed, the missing leader sequence and start codon mutation would be restored from the 3'-terminal redundancy of pgRNA, and then HBeAg could be synthesized. Therefore, HBeAg could be used as a surrogate marker for cccDNA (Zhou, T. et al., Antiviral Res. (2006), 72(2), 116-124; Guo, H. et al., J. Virol. (2007), 81(22), 12472-12484).

HepDES19 cells were seeded at 2 × 10⁶ cells per T150 flask and cultured with the culture medium (Dulbecco's Modified Eagle Medium: Nutrient Mixture F-12 [DMEM-F12, Gibco Cat. 11320-82], 10% Fetal Bovine Serum [FBS, Clontech Cat. 631101], 0.1mM Non-Essential Amino Acids Solution [NEAA, Gibco Cat. 11140-050], 50 µg/mL Penicillin-Streptomycin [PS, Invitrogen Cat. 15140-163], 500 µg/mL Geneticin [G418, Invitrogen Cat. 10131-027]) containing 3µg/mL Tet (Sigma, Cat. 87128) for 5 days. Cells were then seeded at 4 × 10⁶ cells per T150 in the same culture medium as described above in the absence of Tet for 8 days. Cells were then harvested and frozen at density of 2 × 10⁶ cells per mL. For compound testing, the frozen cells were thawed and seeded into 96-well plates at a density of 6 ×10⁴ cells per well. At 24 hours after seeding, half log serial dilutions of compounds made with Dimethyl sulfoxide (DMSO, Sigma, Cat. D2650) were further diluted with the same culture medium as described above before they were added to the cells to reach desired final compound concentrations and 1% DMSO concentration. Plates were then incubated at 37 °C for another 5 days before measurement of HBeAg level and cell viability. Intracellular HBeAg level were measured with enzyme-linked immunosorbent assay (ELISA) kit (Shanghai Kehua Diagnostic Medical Products Co., Ltd). Cell viability was assessed using Cell Counting Kit-8 (Donjindo, Cat. CK04-20). IC₅₀ values were derived from the dose-response curve using 4 parameter logistic curve fit method.

The compounds of the present invention were tested for their capacity to inhibit extracellular HBeAg level as described herein. The compounds of this invention were found to have IC₅₀ below 50 µM. Particular compounds of formula (I) were found to have IC₅₀ below 5.0 µM. Results of HepDES19 primary screen assay are given in Table 1.

**Table 1: Activity data in HepDES19 primary screen assay**

| Example No. | IC₅₀ (µM) | Example No. | IC₅₀ (µM) | Example No. | IC₅₀ (µM) |
|---|---|---|---|---|---|
| **1** | 9.12 | **18** | 14.0 | **37** | 12.2 |
| **2** | 49.8 | **19** | 0.56 | **38** | 2.12 |
| **3** | 30.7 | **20** | 5.68 | **39** | 0.96 |
| **4** | 23.4 | **21** | 47.5 | **40** | 5.90 |
| **5** | 26.0 | **22** | 25.6 | **41** | 19.2 |
| **6** | 21.6 | **23** | 6.88 | **42** | 4.26 |
| **7** | 39.8 | **24** | 46.1 | **43** | 30.1 |
| **8** | 0.23 | **27** | 0.89 | **44** | 6.22 |
| **9** | 2.73 | **28** | 1.08 | **45** | 6.24 |
| **10** | 7.78 | **29** | 2.36 | **46** | 9.84 |
| **11** | 19.0 | **30** | 32.5 | **47** | 3.29 |
| **12** | 0.82 | **31** | 7.13 | **48** | 6.90 |
| **13** | 41.6 | **32** | 10.8 | **49** | 40.1 |
| **14** | 2.07 | **33** | 1.84 | **50** | 26.4 |
| **15** | 10.5 | **34** | 6.01 | **51** | 5.46 |
| **16** | 34.4 | **35** | 46.9 | **52** | 37.7 |
| **17** | 2.40 | **36** | 21.6 | | |

### Example 54: Cryopreserved primary human hepatocytes (PHH) assay

This assay is used to confirm the anti-HBV effect of the compounds in HBV PHH infection assay. Cryopreserved PHH (BioreclamationIVT, Lot YJM) was thawed at 37 °C and gently transferred into pre-warmed InVitroGRO HT medium (BioreclamationIVT, Cat. S03317). The mixture was centrifuged at 70 relative centrifugal force (RCF) for 3 mins at RT, and the supernatant was discarded. Pre-warmed InVitroGRO CP medium (BioreclamationIVT, Cat# S03316) was added to the cell pellet to gently re-suspend cells. The cells were seeded at the density of 5.8 × 10⁴ cells per well to collagen I coated 96-well plate (Gibco, Cat. A1142803) with the InVitroGRO CP medium. All plates were incubated at 37 °C with 5% CO₂ and 85% humidity.

At 20 hours after plating, the medium was changed to PHH culture medium (Dulbecco's Modified Eagle Medium (DMEM)/F12 (1:1) (Gibco, Cat. 11320-033), 10% fetal bovine serum (Gibco Cat. 10099141), 100 U/mL penicillin, 100 µg/mL streptomycin (Gibco, Cat. 151401-122), 5 ng/mL human epidermal growth factor (Invitrogen Cat. PHG0311L), 20 ng/mL dexamethasone (Sigma, Cat. D4902) and 250 ng/mL human recombinant insulin (Gibco, Cat. 12585-014)). And the cells were incubated at 37°C with 5% CO₂ and 85% humidity for 4 hours. The medium was then changed to pre-warmed PHH culture medium containing 4% polyethylene glycol (PEG) MW8000 (Sigma, Cat. P1458-50ML) and 1% DMSO (Sigma, Cat. D2650). 5.8 × 10⁶ genomic equivalents of HBV were added into the medium.

At 24 hours post-infection, the cells were gently washed with PBS and refreshed with PHH culture medium supplemented with 1% DMSO, and 0.25mg/mL Matrix gel (Corning, Cat. 356237) at 200µL per well. All plates were immediately placed in at 37 °C CO₂ incubator.

24 hours later, serial dilutions of compounds made with DMSO were further diluted with the same culture medium (PHH culture medium supplemented with 1% DMSO and 0.25mg/mL Matrix gel as described above) before they were added to the cells to reach desired final compound concentrations and 1% DMSO concentration. The medium containing the compounds were refreshed every three days.

At 9 days post-compound treatment, extracellular HBsAg level were measured with Chemiluminescence Immuno Assay (CLIA) kit (Autobio, HBsAg Quantitative CLIA). Extracellular HBV DNA was extracted by MagNA Pure 96 system (Roche) and then determined by quantitative PCR with the following primers and probe:
HBV-Forward Primer (SEQ ID NO: 1): AAGAAAAACCCCGCCTGTAA (5' to 3');
HBV-Reverse Primer (SEQ ID NO: 2): CCTGTTCTGACTACTGCCTCTCC (5' to 3');
HBV-Probe: 5'+ tetramethylrhodamine + SEQ ID NO: 3 + black hole quencher 2-3', wherein SEQ ID NO: 3 is CCTGATGTGATGTTCTCCATGTTCAGC.

HBsAg IC₅₀ and HBV DNA IC₅₀ values were derived from the dose-response curve using 4 parameter logistic curve fit method. The compounds of formula (I) have HBsAg IC₅₀ < 20 µM, particularly < 1 µM; and HBV DNA IC₅₀ < 50 µM. Test results of the compounds in Cryopreserved PHH assay are given in Table 2.

**Table 2: HBsAg IC₅₀ data in Cryopreserved PHH assay**

| Example No. | HBsAg IC₅₀ (µM) | Example No. | HBsAg IC₅₀ (µM) | Example No. | HBsAg IC₅₀ (µM) |
|---|---|---|---|---|---|
| **1** | 10.6 | **19** | 16.9 | **28** | 0.57 |
| **9** | 3.52 | **20** | 7.40 | **29** | 2.17 |
| **12** | 2.76 | **22** | 3.44 | **35** | 10.5 |
| **14** | 9.85 | **23** | 16.5 | **39** | 5.65 |
| **16** | 3.17 | **25** | 48.4 | **40** | 11.6 |
| **17** | 14.7 | **26** | 18.1 | **41** | 30.7 |
| **18** | 2.84 | **27** | 13.0 | **51** | 5.80 |

## Claims

1. A compound of the formula (I), 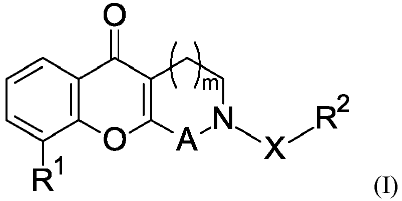 Wherein
R¹ is halogen;
R² is C₁₋₆alkyl, phenyl, phenylC₁₋₆alkyl, pyrazolyl, pyridyl or isoquinolyl; wherein phenyl, phenylC₁₋₆alkyl, pyrazolyl and pyridyl are unsubstituted or substituted by one or two or three substituents independently selected from halogen, C₁₋₆alkyl, haloC₁₋₆alkyl, C₁₋₆alkoxy, haloC₁₋₆alkoxy, C₁₋₆alkoxyC₁₋₆alkyl, C₁₋₆alkoxyC₁₋₆alkoxy, C₁₋₆alkoxycarbonyl, C₁₋₆alkoxyphenylC₁₋₆alkoxy, carboxyC₃₋₇cycloalkoxy, CN and pyrrolidinylcarbonyl;
X is a bond, -C(O)-, -C(O)O- or -C(O)-NH-;
A is -C(R³R⁴)- or -C(O)-;
wherein
R³ is H or C₁₋₆alkyl;
R⁴ is H or C₁₋₆alkyl;
m is 0 or 1;
or a pharmaceutically acceptable salt thereof.

2. A compound according to claim 1, wherein
R¹ is Cl;
R² is butyl, phenyl, phenylmethyl, pyrazolyl, pyridyl or isoquinolyl; wherein phenyl, phenylmethyl, pyrazolyl and pyridyl are unsubstituted or substituted by one or two or three substituents independently selected from F, Cl, Br, CF₃, CN, methyl, methoxy, ethoxy, methoxymethyl, methoxymethoxy, methoxycarbonyl, trifluoromethoxy, carboxycyclobutoxy, pyrrolidinylcarbonyl and methoxyphenylmethoxy;
X is a bond, -C(O)-, -C(O)O- or -C(O)-NH-;
A is -C(R³R⁴)- or -C(O)-;
wherein
R³ is H or methyl;
R⁴ is H or methyl;
m is 0 or 1;
or a pharmaceutically acceptable salt thereof.

3. A compound according to claim 1, or a pharmaceutically acceptable salt thereof, wherein X is a bond.

4. A compound according to claims 1, or a pharmaceutically acceptable salt thereof, wherein A is -CH₂- or -C(O)-.

5. A compound according to any one of claims 1,3 and 4, or a pharmaceutically acceptable salt thereof, wherein R² is phenyl, which is unsubstituted or substituted by one or two substituents independently selected from halogen, haloC₁₋₆alkyl, C₁₋₆alkoxy, C₁₋₆alkoxycarbonyl.

6. A compound according to claims 5, or a pharmaceutically acceptable salt thereof, wherein R² is phenyl, which is unsubstituted or substituted by one or two substituents independently selected from F, Cl, CF₃, methoxy and methoxycarbonyl.

7. A compound according to claim 1, wherein
R¹ is halogen;
R² is phenyl, which is unsubstituted or substituted by one or two substituents independently selected from halogen, haloC₁₋₆alkyl, C₁₋₆alkoxy, C₁₋₆alkoxycarbonyl;
X is a bond;
A is -CH₂- or -C(O)-;
m is 0 or 1;
or a pharmaceutically acceptable salt thereof.

8. A compound according to claim 7, wherein
R¹ is Cl;
R² is phenyl, which is unsubstituted or substituted by one or two substituents independently selected from F, Cl, CF₃, methoxy and methoxycarbonyl;
or a pharmaceutically acceptable salt thereof.

9. A compound according to claim 1 or 2, selected from
tert-butyl 9-chloro-5-oxo-3,4-dihydro-1H-chromeno[2,3-c]pyridine-2(5H)-carboxylate;
2-benzyl-9-chloro-3,4-dihydro-1H-chromeno[2,3-c]pyridin-5(2H)-one;
methyl 3-((9-chloro-5-oxo-3,4-dihydro-1H-chromeno[2,3-c]pyridin-2(5H)-yl)methyl)benzoate;
9-chloro-2-(2-fluorobenzoyl)-3,4-dihydro-1H-chromeno[2,3-c]pyridin-5(2H)-one;
9-chloro-2-[(4-methoxyphenyl)methyl]-3,4-dihydrochromeno[2,3-c]pyridine-1,5-dione;
9-chloro-2-(isoquinoline-1-carbonyl)-3,4-dihydro-1H-chromeno[2,3-c]pyridin-5(2H)-one;
9-chloro-*N*-(2-fluorophenyl)-5-oxo-3,4-dihydro-1H-chromeno[2,3-c]pyridine-2(5H)-carboxamide;
methyl 3-(9-chloro-1,5-dioxo-3,4-dihydro-1H-chromeno[2,3-c]pyridin-2(5H)-yl)benzoate;
9-chloro-2-(4-methoxyphenyl)-3,4-dihydro-1H-chromeno[2,3-c]pyridine-1,5(2H)-dione;
methyl 4-(9-chloro-1,5-dioxo-3,4-dihydro-1H-chromeno[2,3-c]pyridin-2(5H)-yl)benzoate;
3-(4-(9-chloro-1,5-dioxo-3,4-dihydro-1H-chromeno[2,3-c]pyridin-2(5H)-yl)phenoxy)cyclobutanecarboxylic acid;
9-chloro-2-(4-chlorophenyl)-3,4-dihydro-1H-chromeno[2,3-c]pyridine-1,5(2H)-dione;
methyl 6-(9-chloro-5-oxo-3,4-dihydro-1H-chromeno[2,3-c]pyridin-2(5H)-yl)nicotinate;
9-chloro-2-(4-fluorophenyl)-3,4-dihydro-2H-chromeno[2,3-c]pyridine-1,5-dione;
2-(4-bromophenyl)-9-chloro-3,4-dihydro-2H-chromeno[2,3-c]pyridine-1,5-dione;
9-chloro-2-(6-chloropyridin-3-yl)-3,4-dihydro-2H-chromeno[2,3-c]pyridine-1,5-dione;
9-chloro-2-(4-chloro-2-methoxyphenyl)-3,4-dihydro-2H-chromeno[2,3-c]pyridine-1,5-dione;
9-chloro-2-(4-fluoro-3-(trifluoromethyl)phenyl)-3,4-dihydro-2H-chromeno[2,3-c]pyridine-1,5-dione;
9-chloro-2-phenyl-3,4-dihydro-2H-chromeno[2,3-c]pyridine-1,5-dione;
9-chloro-2-(4-(trifluoromethyl)phenyl)-3,4-dihydro-2H-chromeno[2,3-c]pyridine-1,5-dione;
9-chloro-2-(3-(trifluoromethoxy)phenyl)-3,4-dihydro-2H-chromeno[2,3-c]pyridine-1,5-dione;
9-chloro-2-(4-chloro-3-(trifluoromethyl)phenyl)-3,4-dihydro-2H-chromeno[2,3-c]pyridine-1,5-dione;
9-chloro-2-(2-(trifluoromethoxy)phenyl)-3,4-dihydro-2H-chromeno[2,3-c]pyridine-1,5-dione; methyl 2-chloro-5-(9-chloro-1,5-dioxo-1,3,4,5-tetrahydro-2H-chromeno[2,3-c]pyridin-2-yl)benzoate; 3-(2-(9-chloro-1,5-dioxo-1,3,4,5-tetrahydro-2H-chromeno[2,3-c]pyridin-2-yl)phenoxy)cyclobutane-1-carboxylic acid; 3-(5-chloro-2-(9-chloro-1,5-dioxo-1,3,4,5-tetrahydro-2H-chromeno[2,3-c]pyridin-2-yl)phenoxy)cyclobutane-1-carboxylic acid; 9-chloro-2-(4-chloro-2-(trifluoromethyl)phenyl)-3,4-dihydro-2H-chromeno[2,3-c]pyridine-1,5-dione; 9-chloro-2-(3,4-dichlorophenyl)-3,4-dihydro-2H-chromeno[2,3-c]pyridine-1,5-dione; 9-chloro-2-(4-chloro-2-fluorophenyl)-3,4-dihydro-2H-chromeno[2,3-c]pyridine-1,5-dione; 4-(9-chloro-1,5-dioxo-1,3,4,5-tetrahydro-2H-chromeno[2,3-c]pyridin-2-yl)benzonitrile; 9-chloro-2-(2,5-dimethylphenyl)-3,4-dihydro-2H-chromeno[2,3-c]pyridine-1,5-dione; 9-chloro-2-(2-chloro-4-(trifluoromethoxy)phenyl)-3,4-dihydro-2H-chromeno[2,3-c]pyridine-1,5-dione; 9-chloro-2-(4-(trifluoromethoxy)phenyl)-3,4-dihydro-2H-chromeno[2,3-c]pyridine-1,5-dione; 9-chloro-2-(p-tolyl)-3,4-dihydro-2H-chromeno[2,3-c]pyridine-1,5-dione; 9-chloro-2-(1-methyl-1H-pyrazol-4-yl)-3,4-dihydro-2H-chromeno[2,3-c]pyridine-1,5-dione; methyl 5-chloro-2-(9-chloro-1,5-dioxo-1,3,4,5-tetrahydro-2H-chromeno[2,3-c]pyridin-2-yl)benzoate; 5-chloro-2-(9-chloro-1,5-dioxo-1,3,4,5-tetrahydro-2H-chromeno[2,3-c]pyridin-2-yl)benzonitrile; 9-chloro-2-(4-chloro-2-methylphenyl)-3,4-dihydro-2H-chromeno[2,3-c]pyridine-1,5-dione; 9-chloro-2-(4-(trifluoromethyl)phenyl)-l,2,3,4-tetrahydro-5H-chromeno[2,3-c]pyridin-5-one; 9-chloro-2-(4-methoxyphenyl)-1,2,3,4-tetrahydro-5H-chromeno[2,3-c]pyridin-5-one; 9-chloro-2-(3-methoxyphenyl)-1,2,3,4-tetrahydro-5H-chromeno[2,3-c]pyridin-5-one; 9-chloro-2-(4-chlorophenyl)-1,2,3,4-tetrahydro-5H-chromeno[2,3-c]pyridin-5-one; 9-chloro-2-(5-chloro-3-(trifluoromethyl)pyridin-2-yl)-3,4-dihydro-2H-chromeno[2,3-c]pyridine-1,5-dione; 2-(2-bromo-4-chlorophenyl)-9-chloro-3,4-dihydro-2H-chromeno[2,3-c]pyridine-1,5-dione; 9-chloro-2-(5-chloro-3-((4-methoxybenzyl)oxy)pyridin-2-yl)-3,4-dihydro-2H-chromeno[2,3-c]pyridine-1,5-dione; 9-chloro-2-(4-chloro-2-(methoxymethoxy)phenyl)-3,4-dihydro-2H-chromeno[2,3-c]pyridine-1,5-dione;
9-chloro-2-(4-chloro-2-(pyrrolidine-1-carbonyl)phenyl)-3,4-dihydro-2H-chromeno[2,3-c]pyridine-1,5-dione;
9-chloro-2-(4-chloro-2-ethoxyphenyl)-3,4-dihydro-2H-chromeno[2,3-c]pyridine-1,5-dione;
9-chloro-2-(3,4-dichlorophenyl)-1,2,3,4-tetrahydro-5H-chromeno[2,3-c]pyridin-5-one;
9-chloro-2-(4-chloro-2-(methoxymethyl)phenyl)-3,4-dihydro-2H-chromeno[2,3-c]pyridine-1,5-dione;
5-chloro-2-(4-chlorophenyl)-1H-chromeno[2,3-c]pyrrole-3,9-dione; and
5-chloro-2-(4-methoxyphenyl)-1H-chromeno[2,3-c]pyrrole-3,9-dione;
or a pharmaceutically acceptable salt thereof.

10. A compound any one of claims 1 to 8, selected from
methyl 3-(9-chloro-1,5-dioxo-3,4-dihydro-1H-chromeno[2,3-c]pyridin-2(5H)-yl)benzoate;
9-chloro-2-(4-methoxyphenyl)-3,4-dihydro-1H-chromeno[2,3-c]pyridine-1,5(2H)-dione;
9-chloro-2-(4-chlorophenyl)-3,4-dihydro-1H-chromeno[2,3-c]pyridine-1,5(2H)-dione;
9-chloro-2-(4-fluorophenyl)-3,4-dihydro-2H-chromeno[2,3-c]pyridine-1,5-dione;
9-chloro-2-(4-(trifluoromethyl)phenyl)-3,4-dihydro-2H-chromeno[2,3-c]pyridine-1,5-dione;
9-chloro-2-(3,4-dichlorophenyl)-3,4-dihydro-2H-chromeno[2,3-c]pyridine-1,5-dione;
9-chloro-2-(4-chloro-2-fluorophenyl)-3,4-dihydro-2H-chromeno[2,3-c]pyridine-1,5-dione;
9-chloro-2-(4-(trifluoromethyl)phenyl)-1,2,3,4-tetrahydro-5H-chromeno[2,3-c]pyridin-5-one; and
5-chloro-2-(4-chlorophenyl)-1H-chromeno[2,3-c]pyrrole-3,9-dione;
or a pharmaceutically acceptable salt thereof.

11. A process for the preparation of a compound according to any one of claims 1 to 10 comprising
(a) Condensation of compound of formula (VIII), 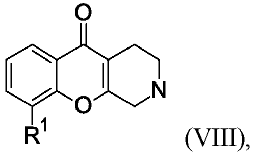 with compound of formula (VIII-1) in the presence of a condensation reagent and a base;
(b) Condensation of compound of formula (VIII) with compound of formula (VIII-2) in the presence of a base;
(c) Condensation of compound of formula (VIII) with compound of formula (VIII-3a) in the presence of a catalyst and a base;
(d) Condensation of compound of formula (VIII) with compound of formula (VIII-3b) in the presence of a base;
(e) Oxidation of compound of formula (IX), 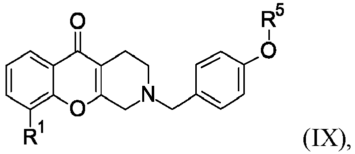 in the presence of an oxidant and a base;
(f) Condensation of compound of formula (X), 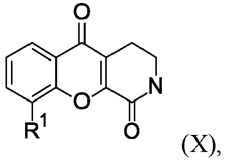 with compound of formula (VIII-3b) in the presence of a catalyst and a base;
(g) Hydrolysis of compound of formula (XI), 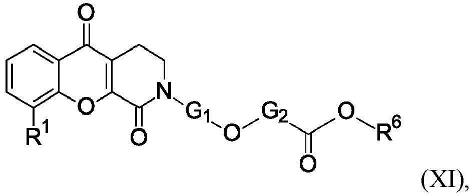 in the presence of an acid;
(h) Hydrolysis of compound of formula (X-5), 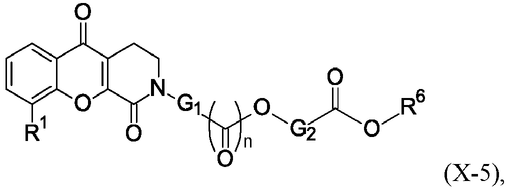 in the presence of an acid;
(i) Condensation of compound of formula (X-3), 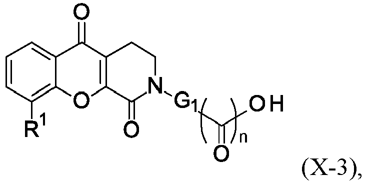 with compound of formula (X-6) in the presence of a condensation reagent and a base;
(j) Condensation of compound of formula (X-3) with compound of formula (X-7) in the presence of a base;
(k) Cyclization of compound of formula (XVI), 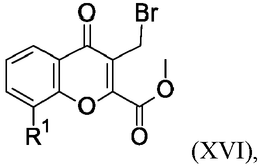 with compound of formula (XVI-1); wherein R¹ and R² are defined as any one of claims 1 to 10; R⁵ is C₁₋₆alkyl; R⁶ is C₁₋₆alkyl; G₁ is phenyl, which is unsubstituted or substituted by halogen; G₂ is C₃₋₇cycloalkyl; n is 0 or 1.

12. A compound according to any one of claims 1 to 10 for use as therapeutically active substance.

13. A pharmaceutical composition comprising a compound in accordance with any one of claims 1 to 10 and a therapeutically inert carrier.

14. A compound according to any one of claims 1 to 10 for use in the treatment or prophylaxis of HBV infection.

## Patentansprüche

1. Verbindung der Formel (I) 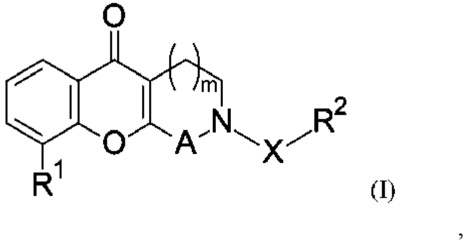 wobei
R¹ Halogen ist;
R² C₁₋₆-Alkyl, Phenyl, Phenyl-C₁₋₆-alkyl, Pyrazolyl, Pyridyl oder Isochinolyl ist; wobei Phenyl, Phenyl-C₁₋₆-alkyl, Pyrazolyl und Pyridyl unsubstituiert oder mit einem oder zwei oder drei Substituenten substituiert sind, die unabhängig voneinander aus Halogen, C₁₋₆-Alkyl, Halogen-C₁₋₆-alkyl, C₁₋₆-Alkoxy, Halogen-C₁₋₆-alkoxy, C₁₋₆-Alkoxy-C₁₋₆-alkyl, C₁₋₆-Alkoxy-C₁₋₆-alkoxy, C₁₋₆-Alkoxycarbonyl, C₁₋₆-Alkoxyphenyl-C₁₋₆-alkoxy, Carboxy-C₃₋₇-cycloalkoxy, CN und Pyrrolidinylcarbonyl ausgewählt sind;
X eine Bindung, -C(O)-, -C(O)O- oder -C(O)-NH- ist;
A -C(R³R⁴)- oder -C(O)- ist;
wobei
R³ H oder C₁₋₆-Alkyl ist;
R⁴ H oder C₁₋₆-Alkyl ist;
m 0 oder 1 ist;
oder ein pharmazeutisch unbedenkliches Salz davon.

2. Verbindung nach Anspruch 1, wobei
R¹ Cl ist;
R² Butyl, Phenyl, Phenylmethyl, Pyrazolyl, Pyridyl oder Isochinolyl ist; wobei Phenyl, Phenylmethyl, Pyrazolyl und Pyridyl unsubstituiert oder mit einem oder zwei oder drei
X Substituenten substituiert sind, die unabhängig voneinander aus F, Cl, Br, CF₃, CN, Methyl, Methoxy, Ethoxy, Methoxymethyl, Methoxymethoxy, Methoxycarbonyl, Trifluormethoxy, Carboxycyclobutoxy, Pyrrolidinylcarbonyl und Methoxyphenylmethoxy ausgewählt sind; eine Bindung, -C(O)-, -C(O)O- oder -C(O)-NH- ist;
A -C(R³R⁴)- oder -C(O)- ist;
wobei
R³ H oder Methyl ist;
R⁴ H oder Methyl ist;
m 0 oder 1 ist;
oder ein pharmazeutisch unbedenkliches Salz davon.

3. Verbindung nach Anspruch 1 oder ein pharmazeutisch unbedenkliches Salz davon, wobei X eine Bindung ist.

4. Verbindung nach Anspruch 1 oder ein pharmazeutisch unbedenkliches Salz davon, wobei A -CH₂- oder -C(O)- ist.

5. Verbindung nach einem der Ansprüche 1, 3 und 4 oder ein pharmazeutisch unbedenkliches Salz davon, wobei R² Phenyl ist, das unsubstituiert oder mit einem oder zwei Substituenten substituiert ist, die unabhängig voneinander aus Halogen, Halogen-C₁₋₆-alkyl, C₁₋₆-Alkoxy, C₁₋₆-Alkoxycarbonyl ausgewählt sind.

6. Verbindung nach Anspruch 5 oder ein pharmazeutisch unbedenkliches Salz davon, wobei R² Phenyl ist, das unsubstituiert oder mit einem oder zwei Substituenten substituiert ist, die unabhängig voneinander aus F, Cl, CF₃, Methoxy und Methoxycarbonyl ausgewählt sind.

7. Verbindung nach Anspruch 1, wobei
R¹ Halogen ist;
R² Phenyl ist, das unsubstituiert oder mit einem oder zwei Substituenten substituiert ist, die unabhängig voneinander aus Halogen, Halogen-C₁₋₆-alkyl, C₁₋₆-Alkoxy, C₁₋₆-Alkoxycarbonyl ausgewählt sind;
X eine Bindung ist;
A -CH₂- oder -C(O)- ist;
m 0 oder 1 ist;
oder ein pharmazeutisch unbedenkliches Salz davon.

8. Verbindung nach Anspruch 7, wobei
R¹ Cl ist;
R² Phenyl ist, das unsubstituiert oder mit einem oder zwei Substituenten substituiert ist, die unabhängig voneinander aus F, Cl, CF₃, Methoxy und Methoxycarbonyl ausgewählt sind;
oder ein pharmazeutisch unbedenkliches Salz davon.

9. Verbindung nach Anspruch 1 oder 2, ausgewählt aus
tert-Butyl-9-chlor-5-oxo-3,4-dihydro-1H-chromeno[2,3-c]pyridin-2(SH)-carboxylat;
2-Benzyl-9-chlor-3,4-dihydro-1H-chromeno[2,3-c]pyridin-5(2H)-on;
Methyl-3-((9-chlor-5-oxo-3,4-dihydro-1H-chromeno[2,3-c]pyridin-2(5H)-yl)methyl)benzoat;
9-Chlor-2-(2-fluorbenzoyl)-3,4-dihydro-1H-chromeno[2,3-c]pyridin-5(2H)-on;
9-Chlor-2-[(4-methoxyphenyl)methyl]-3,4-dihydrochromeno[2,3-c]pyridin-1,5-dion;
9-Chlor-2-(isochinolin-1-carbonyl)-3,4-dihydro-1H-chromeno[2,3-c]pyridin-5(2H)-on;
9-Chlor-N (2-fluorphenyl)-5-oxo-3,4-dihydro-1H-chromeno[2,3-c]pyridin-2(SH)-carboxamid;
Methyl-3-(9-chlor-1,5-dioxo-3,4-dihydro-1H-chromeno[2,3-c]pyridin-2(SH)-yl)benzoat;
9-Chlor-2-(4-methoxyphenyl)-3,4-dihydro-1H-chromeno[2,3-c]pyridin-1,5(2H)-dion;
Methyl-4-(9-chlor-1,5-dioxo-3,4-dihydro-1H-chromeno[2,3-c]pyridin-2(5H)-yl)benzoat;
3-(4-(9-Chlor-1, 5-dioxo-3,4-dihydro-1H-chromeno[2,3-c]pyridin-2(SH)-yl)phenoxy)cyclobutancarbonsäure;
9-Chlor-2-(4-chlorphenyl)-3,4-dihydro-1H-chromeno[2,3-c]pyridin-1,5(2H)-dion;
Methyl-6-(9-chlor-5-oxo-3,4-dihydro-1H-chromeno[2,3-c]pyridin-2(SH)-yl)nicotinat;
9-Chlor-2-(4-fluorphenyl)-3,4-dihydro-2H-chromeno[2,3-c]pyridin-1,5-dion;
2-(4-Bromphenyl)-9-chlor-3,4-dihydro-2H-chromeno[2,3-c]pyridin-1,5-dion;
9-Chlor-2-(6-chlorpyridin-3-yl)-3,4-dihydro-2H-chromeno[2,3-c]pyridin-1,5-dion;
9-Chlor-2-(4-chlor-2-methoxyphenyl)-3,4-dihydro-2H-chromeno[2,3-c]pyridin-1,5-dion;
9-Chlor-2-(4-fluor-3-(trifluormethyl)phenyl)-3,4-dihydro-2H-chromeno[2,3-c]pyridin-1,5-dion;
9-Chlor-2-phenyl-3,4-dihydro-2H-chromeno[2,3-c]pyridin-1,5-dion;
9-Chlor-2-(4-(trifluormethyl)phenyl)-3,4-dihydro-2H-chromeno[2,3-c]pyridin-1,5-dion;
9-Chlor-2-(3-(trifluormethoxy)phenyl)-3,4-dihydro-2H-chromeno[2,3-c]pyridin-1,5-dion;
9-Chlor-2-(4-chlor-3-(trifluormethyl)phenyl)-3,4-dihydro-2H-chromeno[2,3-c]pyridin-1,5-dion;
9-Chlor-2-(2-(trifluormethoxy)phenyl)-3,4-dihydro-2H-chromeno[2,3-c]pyridin-1, 5-dion; Methyl-2-chlor-5-(9-chlor-1,5-dioxo-1,3,4,5-tetrahydro-2H-chromeno[2,3-c]pyridin-2-yl)benzoat; 3 -(2-(9-Chlor-1,5-dioxo-1,3,4,5-tetrahydro-2H-chromeno[2,3 -c]pyridin-2-yl)phenoxy)cyclobutan-1 -carbonsäure; 3-(5-Chlor-2-(9-chlor-1,5-dioxo-1,3,4,5-tetrahydro-2H-chromeno[2,3-c]pyridin-2-yl)phenoxy)cyclobutan-1 -carbonsäure; 9-Chlor-2-(4-chlor-2-(trifluormethyl)phenyl)-3,4-dihydro-2H-chromeno[2,3-c]pyridin-1,5-dion; 9-Chlor-2-(3,4-dichlorphenyl)-3,4-dihydro-2H-chromeno[2,3-c]pyridin-1,5-dion; 9-Chlor-2-(4-chlor-2-fluorphenyl)-3,4-dihydro-2H-chromeno[2,3-c]pyridin-1,5-dion; 4-(9-Chlor-1,5-dioxo-1,3,4,5-tetrahydro-2H-chromeno[2,3-c]pyridin-2-yl)benzonitril; 9-Chlor-2-(2,5-dimethylphenyl)-3,4-dihydro-2H-chromeno[2,3-c]pyridin-1,5-dion; 9-Chlor-2-(2-chlor-4-(trifluormethoxy)phenyl)-3,4-dihydro-2H-chromeno[2,3-c]pyridin-1,5-dion; 9-Chlor-2-(4-(trifluormethoxy)phenyl)-3,4-dihydro-2H-chromeno[2,3-c]pyridin-1,5-dion; 9-Chlor-2-(p-tolyl)-3,4-dihydro-2H-chromeno[2,3-c]pyridin-1,5-dion; 9-Chlor-2-(1-methyl-1H-pyrazol-4-yl)-3,4-dihydro-2H-chromeno[2,3-c]pyridin-1,5-dion; Methyl-5-chlor-2-(9-chlor-1,5-dioxo-1,3,4,5-tetrahydro-2H-chromeno[2,3-c]pyridin-2-yl)benzoat; 5-Chlor-2-(9-chlor-1,5-dioxo-1,3,4,5-tetrahydro-2H-chromeno[2,3-c]pyridin-2-yl)benzonitril; 9-Chlor-2-(4-chlor-2-methylphenyl)-3,4-dihydro-2H-chromeno[2,3-c]pyridin-1,5-dion; 9-Chlor-2-(4-(trifluormethyl)phenyl)-1,2,3,4-tetrahydro-5H-chromeno[2,3-c]pyridin-5-on; 9-Chlor-2-(4-methoxyphenyl)-1,2,3,4-tetrahydro-5H-chromeno[2,3-c]pyridin-5-on; 9-Chlor-2-(3-methoxyphenyl)-1,2,3,4-tetrahydro-5H-chromeno[2,3-c]pyridin-5-on; 9-Chlor-2-(4-chlorphenyl)-1,2,3,4-tetrahydro-5H-chromeno[2,3-c]pyridin-5-on; 9-Chlor-2-(5-chlor-3-(trifluormethyl)pyridin-2-yl)-3,4-dihydro-2H-chromeno[2,3-c]pyridin-1,5-dion; 2-(2-Brom-4-chlorphenyl)-9-chlor-3,4-dihydro-2H-chromeno[2,3-c]pyridin-1,5-dion; 9-Chlor-2-(5-chlor-3-((4-methoxybenzyl)oxy)pyridin-2-yl)-3,4-dihydro-2H-chromeno[2,3-c]pyridin-1,5-dion, 9-Chlor-2-(4-chlor-2-(methoxymethoxy)phenyl)-3,4-dihydro-2H-chromeno[2,3-c]pyridin-1,5-dion; 9-Chlor-2-(4-chlor-2-(pyrrolidin-1-carbonyl)phenyl)-3,4-dihydro-2H-chromeno[2,3-c]pyridin-1,5-dion,
9-Chlor-2-(4-chlor-2-ethoxyphenyl)-3,4-dihydro-2H-chromeno[2,3-c]pyridin-1,5-dion;
9-Chlor-2-(3,4-dichlorphenyl)-1,2,3,4-tetrahydro-5H-chromeno[2,3-c]pyridin-5-on;
9-Chlor-2-(4-chlor-2-(methoxymethyl)phenyl)-3,4-dihydro-2H-chromeno[2,3-c]pyridin-1,5-dion;
5-Chlor-2-(4-chlorphenyl)-1H-chromeno[2,3-c]pyrrol-3,9-dion und
5-Chlor-2-(4-methoxyphenyl)-1H-chromeno[2,3-c]pyrrol-3,9-dion;
oder ein pharmazeutisch unbedenkliches Salz davon.

10. Verbindung nach einem der Ansprüche 1 bis 8, ausgewählt aus
Methyl-3-(9-chlor-1,5-dioxo-3,4-dihydro-1H-chromeno[2,3-c]pyridin-2(5H)-yl)benzoat;
9-Chlor-2-(4-methoxyphenyl)-3,4-dihydro-1H-chromeno[2,3-c]pyridin-1,5(2H)-dion;
9-Chlor-2-(4-chlorphenyl)-3,4-dihydro-1H-chromeno[2,3-c]pyridin-1,5(2H)-dion;
9-Chlor-2-(4-fluorphenyl)-3,4-dihydro-2H-chromeno[2,3-c]pyridin-1,5-dion;
9-Chlor-2-(4-(trifluormethyl)phenyl)-3,4-dihydro-2H-chromeno[2,3-c]pyridin-1,5-dion;
9-Chlor-2-(3,4-dichlorphenyl)-3,4-dihydro-2H-chromeno[2,3-c]pyridin-1,5-dion;
9-Chlor-2-(4-chlor-2-fluorphenyl)-3,4-dihydro-2H-chromeno[2,3-c]pyridin-1,5-dion,
9-Chlor-2-(4-(trifluormethyl)phenyl)-1,2,3,4-tetrahydro-5H-chromeno[2,3-c]pyridin-5-on; und
5-Chlor-2-(4-chlorphenyl)-1H-chromeno[2,3-c]pyrrol-3,9-dion;
oder ein pharmazeutisch unbedenkliches Salz davon.

11. Verfahren zur Herstellung einer Verbindung nach einem der Ansprüche 1 bis 10, umfassend
(a) Kondensation einer Verbindung der Formel (VIII) 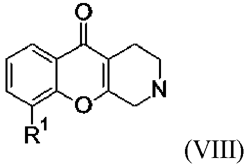 mit einer Verbindung der Formel (VIII-1) in der Gegenwart eines Kondensationsreagens und einer Base;
(b) Kondensation einer Verbindung der Formel (VIII) mit einer Verbindung der Formel (VIII-2) in der Gegenwart einer Base;
(c) Kondensation einer Verbindung der Formel (VIII) mit einer Verbindung der Formel (VIII-3a) in der Gegenwart eines Katalysators und einer Base;
(d) Kondensation einer Verbindung der Formel (VIII) mit einer Verbindung der Formel (VIII-3b) in der Gegenwart einer Base;
(e) Oxidation einer Verbindung der Formel (IX) 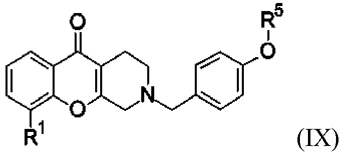 in der Gegenwart eines Oxidationsmittels und einer Base;
(f) Kondensation einer Verbindung der Formel (X) 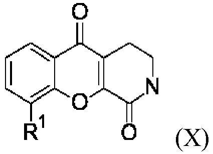 mit einer Verbindung der Formel (VIII-3b) in der Gegenwart eines Katalysators und einer Base;
(g) Hydrolyse einer Verbindung der Formel (XI) 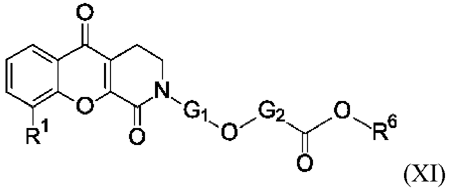 in der Gegenwart einer Säure;
(h) Hydrolyse einer Verbindung der Formel (X-5) 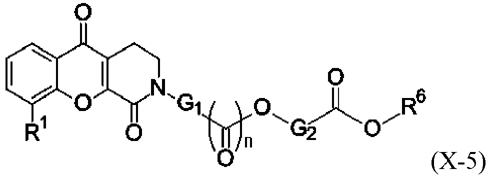 in der Gegenwart einer Säure;
(i) Kondensation einer Verbindung der Formel (X-3) 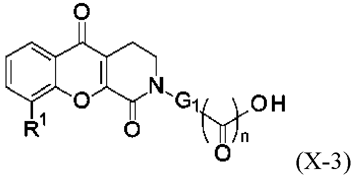 mit einer Verbindung der Formel (X-6) in der Gegenwart eines Kondensationsreagens und einer Base;
(j) Kondensation einer Verbindung der Formel (X-3) mit einer Verbindung der Formel (X-7) in der Gegenwart einer Base;
(k) Cyclisierung einer Verbindung der Formel (XVI) 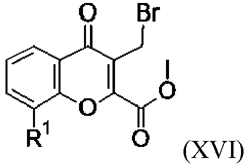 mit einer Verbindung der Formel (XVI-1); wobei R¹ und R² wie in einem der Ansprüche 1 bis 10 definiert sind; R⁵ C₁₋₆-Alkyl ist;
R⁶ C₁₋₆-Alkyl ist; G₁ Phenyl ist, das unsubstituiert oder mit Halogen substituiert ist; G₂ C₃₋₇-Cycloalkyl ist; n 0 oder 1 ist.

12. Verbindung nach einem der Ansprüche 1 bis 10 zur Verwendung als therapeutisch wirksame Substanz.

13. Pharmazeutische Zusammensetzung, umfassend eine Verbindung nach einem der Ansprüche 1 bis 10 und einen therapeutisch inerten Träger.

14. Verbindung nach einem der Ansprüche 1 bis 10 zur Verwendung bei der Behandlung oder Prophylaxe einer HBV-Infektion.

## Revendications

1. Composé de formule (I), 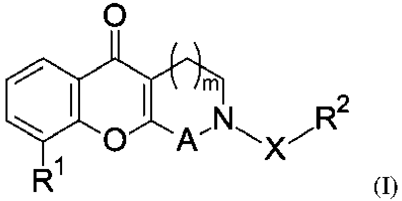 dans lequel
R¹ représente un halogène ;
R² représente un alkyle en C₁₋₆, un phényle, un phénylalkyle en C₁₋₆, un pyrazolyle, un pyridyle ou un isoquinoléyle ; dans lequel le phényle, le phénylalkyle en C₁₋₆, le pyrazolyle et le pyridyle sont non substitués ou substitués par un ou deux ou trois substituants indépendamment choisis parmi un halogène, un alkyle en C₁₋₆, un halogénoalkyle en C₁₋₆, un alcoxy en C₁₋₆, un halogénoalcoxy en C₁₋₆, un alcoxy en C₁₋₆-alkyle en C₁₋₆, un alcoxy en C₁₋₆-alcoxy en C₁₋₆, un alcoxy en C₁₋₆-carbonyle, un alcoxy en C₁₋₆-phénylalcoxy en C₁₋₆, un carboxycycloalcoxy en C₃₋₇, CN et un pyrrolidinylcarbonyle ;
X représente une liaison, -C(O)-, -C(O)O- ou -C(O)-NH- ;
A représente -C(R³R⁴)- ou -C(O)- ;
dans lequel
R³ représente H ou un alkyle en C₁₋₆ ;
R⁴ représente H ou un alkyle en C₁₋₆ ;
m représente 0 ou 1 ;
ou un sel pharmaceutiquement acceptable de celui-ci.

2. Composé selon la revendication 1, dans lequel
R¹ représente Cl ;
R² représente un butyle, un phényle, un phénylméthyle, un pyrazolyle, un pyridyle ou un isoquinoléyle ; dans lequel le phényle, le phénylméthyle, le pyrazolyle et le pyridyle sont non substitués ou substitués par un ou deux ou trois substituants indépendamment choisis parmi F, Cl, Br, CF₃, CN, un méthyle, un méthoxy, un éthoxy, un méthoxyméthyle, un méthoxyméthoxy, un méthoxycarbonyle, un trifluorométhoxy, un carboxycyclobutoxy, un pyrrolidinylcarbonyle et un méthoxyphénylméthoxy ;
X représente une liaison, -C(O)-, -C(O)O- ou -C(O)-NH- ;
A représente -C(R³R⁴)- ou -C(O)- ;
dans lequel
R³ représente H ou un méthyle ;
R⁴ représente H ou un méthyle ;
m représente 0 ou 1 ;
ou un sel pharmaceutiquement acceptable de celui-ci.

3. Composé selon la revendication 1, ou un sel pharmaceutiquement acceptable de celui-ci, dans lequel X représente une liaison.

4. Composé selon les revendications 1, ou un sel pharmaceutiquement acceptable de celui-ci, dans lequel A représente -CH₂- ou -C(O)-.

5. Composé selon l'une quelconque des revendications 1, 3 et 4, ou un sel pharmaceutiquement acceptable de celui-ci, dans lequel R² représente un phényle, qui est non substitué ou substitué par un ou deux substituants indépendamment choisis parmi un halogène, un halogénoalkyle en C₁₋₆, un alcoxy en C₁₋₆, un alcoxy en C₁₋₆-carbonyle.

6. Composé selon la revendication 5, ou un sel pharmaceutiquement acceptable de celui-ci, dans lequel R² représente un phényle, qui est non substitué ou substitué par un ou deux substituants indépendamment choisis parmi F, Cl, CF₃, un méthoxy et un méthoxycarbonyle.

7. Composé selon la revendication 1, dans lequel
R¹ représente un halogène ;
R² représente un phényle, qui est non substitué ou substitué par un ou deux substituants indépendamment choisis parmi un halogène, un halogénoalkyle en C₁₋₆, un alcoxy en C₁₋₆, un alcoxy en C₁₋₆-carbonyle ;
X représente une liaison ;
A représente -CH₂- ou -C(O)- ;
m représente 0 ou 1 ;
ou un sel pharmaceutiquement acceptable de celui-ci.

8. Composé selon la revendication 7, dans lequel
R¹ représente Cl ;
R² représente un phényle, qui est non substitué ou substitué par un ou deux substituants indépendamment choisis parmi F, Cl, CF₃, un méthoxy et un méthoxycarbonyle ;
ou un sel pharmaceutiquement acceptable de celui-ci.

9. Composé selon la revendication 1 ou 2, choisi parmi
le 9-chloro-5-oxo-3,4-dihydro-1H-chroméno[2,3-c]pyridine-2(5H)-carboxylate de *tert*-butyle ;
la 2-benzyl-9-chloro-3,4-dihydro-1H-chroméno[2,3-c]pyridin-5(2H)-one ;
le 3-((9-chloro-5-oxo-3,4-dihydro-1H-chroméno[2,3-c]pyridin-2(5H)-yl)méthyl)benzoate de méthyle ;
la 9-chloro-2-(2-fluorobenzoyl)-3,4-dihydro-1H-chroméno[2,3-c]pyridin-5(2H)-one ;
la 9-chloro-2-[(4-méthoxyphényl)méthyl]-3,4-dihydrochroméno[2,3-c]pyridine-1,5-dione ;
la 9-chloro-2-(isoquinoléine-1-carbonyl)-3,4-dihydro-1H-chroméno[2,3-c]pyridin-5(2H)-one ;
le 9-chloro-N-(2-fluorophényl)-5-oxo-3,4-dihydro-1H-chroméno[2,3-c]pyridine-2(5H)-carboxamide ;
le 3-(9-chloro-1,5-dioxo-3,4-dihydro-1H-chroméno[2,3-c]pyridin-2(5H)-yl)benzoate de méthyle ;
la 9-chloro-2-(4-méthoxyphényl)-3,4-dihydro-1H-chroméno[2,3-c]pyridine-1,5(2H)-dione ;
le 4-(9-chloro-1,5-dioxo-3,4-dihydro-1H-chroméno[2,3-c]pyridin-2(5H)-yl)benzoate de méthyle ;
l'acide 3-(4-(9-chloro-1,5-dioxo-3,4-dihydro-1H-chroméno[2,3-c]pyridin-2(5H)-yl)phénoxy)cyclobutanecarboxylique ;
la 9-chloro-2-(4-chlorophényl)-3,4-dihydro-1H-chroméno[2,3-c]pyridine-1,5(2H)-dione ;
le 6-(9-chloro-5-oxo-3,4-dihydro-1H-chroméno[2,3-c]pyridin-2(5H)-yl)nicotinate de méthyle ;
la 9-chloro-2-(4-fluorophényl)-3,4-dihydro-2H-chroméno[2,3-c]pyridine-1,5-dione ;
la 2-(4-bromophényl)-9-chloro-3,4-dihydro-2H-chroméno[2,3-c]pyridine-1,5-dione ;
la 9-chloro-2-(6-chloropyridin-3-yl)-3,4-dihydro-2H-chroméno[2,3-c]pyridine-1,5-dione ;
la 9-chloro-2-(4-chloro-2-méthoxyphényl)-3,4-dihydro-2H-chroméno[2,3-c]pyridine-1,5-dione ;
la 9-chloro-2-(4-fluoro-3-(trifluorométhyl)phényl)-3,4-dihydro-2H-chroméno[2,3-c]pyridine-1,5-dione ;
la 9-chloro-2-phényl-3,4-dihydro-2H-chroméno[2,3-c]pyridine-1,5-dione ;
la 9-chloro-2-(4-(trifluorométhyl)phényl)-3,4-dihydro-2H-chroméno[2,3-c]pyridine-1,5-dione ;
la 9-chloro-2-(3-(trifluorométhoxy)phényl)-3,4-dihydro-2H-chroméno[2,3-c]pyridine-1,5-dione ;
la 9-chloro-2-(4-chloro-3-(trifluorométhyl)phényl)-3,4-dihydro-2H-chroméno[2,3-c]pyridine-1,5-dione ;
la 9-chloro-2-(2-(trifluorométhoxy)phényl)-3,4-dihydro-2H-chroméno[2,3-c]pyridine-1,5-dione ;
le 2-chloro-5-(9-chloro-1,5-dioxo-1,3,4,5-tétrahydro-2H-chroméno[2,3-c]pyridin-2-yl)benzoate de méthyle ;
l'acide 3-(2-(9-chloro-1,5-dioxo-1,3,4,5-tétrahydro-2H-chroméno[2,3-c]pyridin-2-yl)phénoxy)cyclobutane-1-carboxylique ;
l'acide 3-(5-chloro-2-(9-chloro-1,5-dioxo-1,3,4,5-tétrahydro-2H-chroméno[2,3-c]pyridin-2-yl)phénoxy)cyclobutane-1-carboxylique ;
la 9-chloro-2-(4-chloro-2-(trifluorométhyl)phényl)-3,4-dihydro-2H-chroméno[2,3-c]pyridine-1,5-dione ;
la 9-chloro-2-(3,4-dichlorophényl)-3,4-dihydro-2H-chroméno[2,3-c]pyridine-1,5-dione ;
la 9-chloro-2-(4-chloro-2-fluorophényl)-3,4-dihydro-2H-chroméno[2,3-c]pyridine-1,5-dione ;
le 4-(9-chloro-1,5-dioxo-1,3,4,5-tétrahydro-2H-chroméno[2,3-c]pyridin-2-yl)benzonitrile ;
la 9-chloro-2-(2,5-diméthylphényl)-3,4-dihydro-2H-chroméno[2,3-c]pyridine-1,5-dione ;
la 9-chloro-2-(2-chloro-4-(trifluorométhoxy)phényl)-3,4-dihydro-2H-chroméno[2,3-c]pyridine-1,5-dione ;
la 9-chloro-2-(4-(trifluorométhoxy)phényl)-3,4-dihydro-2H-chroméno[2,3-c]pyridine-1,5-dione ;
la 9-chloro-2-(p-tolyl)-3,4-dihydro-2H-chroméno[2,3-c]pyridine-1,5-dione ;
la 9-chloro-2-(1-méthyl-1H-pyrazol-4-yl)-3,4-dihydro-2H-chroméno[2,3-c]pyridine-1,5-dione ;
le 5-chloro-2-(9-chloro-1,5-dioxo-1,3,4,5-tétrahydro-2H-chroméno[2,3-c]pyridin-2-yl)benzoate de méthyle ;
le 5-chloro-2-(9-chloro-1,5-dioxo-1,3,4,5-tétrahydro-2H-chroméno[2,3-c]pyridin-2-yl)benzonitrile ;
la 9-chloro-2-(4-chloro-2-méthylphényl)-3,4-dihydro-2H-chroméno[2,3-c]pyridine-1,5-dione ;
la 9-chloro-2-(4-(trifluorométhyl)phényl)-1,2,3,4-tétrahydro-5H-chroméno[2,3-c]pyridin-5-one ;
la 9-chloro-2-(4-méthoxyphényl)-1,2,3,4-tétrahydro-5H-chroméno[2,3-c]pyridin-5-one ;
la 9-chloro-2-(3-méthoxyphényl)-1,2,3,4-tétrahydro-5H-chroméno[2,3-c]pyridin-5-one ;
la 9-chloro-2-(4-chlorophényl)-1,2,3,4-tétrahydro-5H-chroméno[2,3-c]pyridin-5-one ;
la 9-chloro-2-(5-chloro-3-(trifluorométhyl)pyridin-2-yl)-3,4-dihydro-2H-chroméno[2,3-c]pyridine-1,5-dione ;
la 2-(2-bromo-4-chlorophényl)-9-chloro-3,4-dihydro-2H-chroméno[2,3-c]pyridine-1,5-dione ;
la 9-chloro-2-(5-chloro-3-((4-méthoxybenzyl)oxy)pyridin-2-yl)-3,4-dihydro-2H-chroméno[2,3-c]pyridine-1,5-dione ;
la 9-chloro-2-(4-chloro-2-(méthoxyméthoxy)phényl)-3,4-dihydro-2H-chroméno[2,3-c]pyridine-1,5-dione ;
la 9-chloro-2-(4-chloro-2-(pyrrolidine-1-carbonyl)phényl)-3,4-dihydro-2H-chroméno[2,3-c]pyridine-1,5-dione ;
la 9-chloro-2-(4-chloro-2-éthoxyphényl)-3,4-dihydro-2H-chroméno[2,3-c]pyridine-1,5-dione ;
la 9-chloro-2-(3,4-dichlorophényl)-1,2,3,4-tétrahydro-5H-chroméno[2,3-c]pyridin-5-one ;
la 9-chloro-2-(4-chloro-2-(méthoxyméthyl)phényl)-3,4-dihydro-2H-chroméno[2,3-c]pyridine-1,5-dione;
la 5-chloro-2-(4-chlorophényl)-1H-chroméno[2,3-c]pyrrole-3,9-dione ; et
la 5-chloro-2-(4-méthoxyphényl)-1H-chroméno[2,3-c]pyrrole-3,9-dione ;
ou un sel pharmaceutiquement acceptable de ceux-ci.

10. Composé selon l'une quelconque des revendications 1 à 8, choisi parmi
le 3-(9-chloro-1,5-dioxo-3,4-dihydro-1H-chroméno[2,3-c]pyridin-2(5H)-yl)benzoate de méthyle ;
la 9-chloro-2-(4-méthoxyphényl)-3,4-dihydro-1H-chroméno[2,3-c]pyridine-1,5(2H)-dione ;
la 9-chloro-2-(4-chlorophényl)-3,4-dihydro-1H-chroméno[2,3-c]pyridine-1,5(2H)-dione ;
la 9-chloro-2-(4-fluorophényl)-3,4-dihydro-2H-chroméno[2,3-c]pyridine-1,5-dione ;
la 9-chloro-2-(4-(trifluorométhyl)phényl)-3,4-dihydro-2H-chroméno[2,3-c]pyridine-1,5-dione ;
la 9-chloro-2-(3,4-dichlorophényl)-3,4-dihydro-2H-chroméno[2,3-c]pyridine-1,5-dione ;
la 9-chloro-2-(4-chloro-2-fluorophényl)-3,4-dihydro-2H-chroméno[2,3-c]pyridine-1,5-dione ;
la 9-chloro-2-(4-(trifluorométhyl)phényl)-1,2,3,4-tétrahydro-5H-chroméno[2,3-c]pyridin-5-one ; et
la 5-chloro-2-(4-chlorophényl)-1H-chroméno[2,3-c]pyrrole-3,9-dione ;
ou un sel pharmaceutiquement acceptable de ceux-ci.

11. Procédé de préparation d'un composé selon l'une quelconque des revendications 1 à 10 comprenant
(a) la condensation d'un composé de formule (VIII), 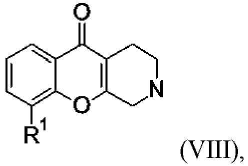 avec un composé de formule (VIII-1) en présence d'un réactif de condensation et d'une base ;
(b) la condensation d'un composé de formule (VIII) avec un composé de formule (VIII-2) en présence d'une base ;
(c) la condensation d'un composé de formule (VIII) avec un composé de formule (VIII-3a) en présence d'un catalyseur et d'une base ;
(d) la condensation d'un composé de formule (VIII) avec un composé de formule (VIII-3b) en présence d'une base ;
(e) l'oxydation d'un composé de formule (IX), 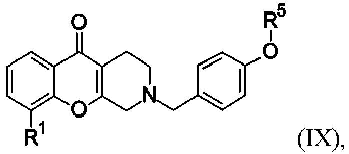 en présence d'un oxydant et d'une base ;
(f) la condensation d'un composé de formule (X), 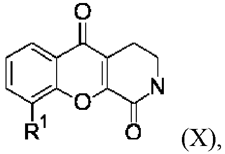 avec un composé de formule (VIII-3b) en présence d'un catalyseur et d'une base ;
(g) l'hydrolyse d'un composé de formule (XI), 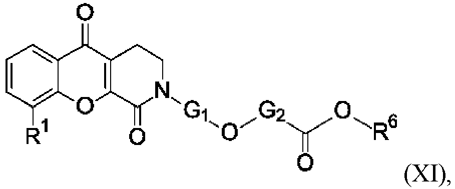 en présence d'un acide ;
(h) l'hydrolyse d'un composé de formule (X-5), 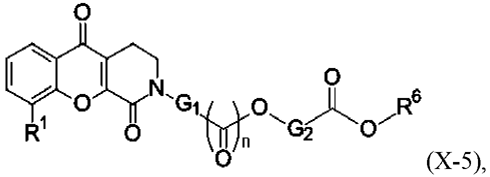 en présence d'un acide ;
(i) la condensation d'un composé de formule (X-3), 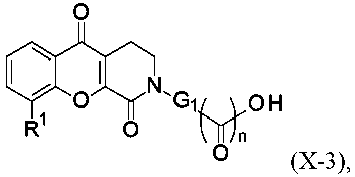 avec un composé de formule (X-6) en présence d'un réactif de condensation et d'une base ;
(j) la condensation d'un composé de formule (X-3) avec un composé de formule (X-7) en présence d'une base ;
(k) la cyclisation d'un composé de formule (XVI), 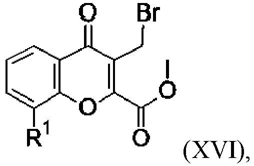 avec un composé de formule (XVI-1) ;
dans lequel R¹ et R² sont tels que définis dans l'une quelconque des revendications 1 à 10 ; R⁵ représente un alkyle en C₁₋₆ ; R⁶ représente un alkyle en C₁₋₆ ; G₁ représente un phényle, qui est non substitué ou substitué par un halogène ; G₂ représente un cycloalkyle en C₃₋₇ ; n représente 0 ou 1.

12. Composé selon l'une quelconque des revendications 1 à 10 pour une utilisation comme substance thérapeutiquement active.

13. Composition pharmaceutique comprenant un composé selon l'une quelconque des revendications 1 à 10 et un véhicule thérapeutiquement inerte.

14. Composé selon l'une quelconque des revendications 1 à 10 pour une utilisation dans le traitement ou la prophylaxie d'une infection par le VHB.
